# EUROPEAN PATENT APPLICATION

(11) **EP 1 793 005 A1**
(43) Date of publication of application: **06.06.2007**
(21) Application number: 05785873.0
(22) Date of filing: 14.09.2005
(51) Int. Cl.: C12Q 1/68, C12N 15/09, G01N 33/50, A61K 39/395, A61P 25/16, A61P 25/18, A61P 25/24

(54) **NOVEL USE OF PrPP AND RECEPTOR THEREOF**

(30) Priority: 15.09.2004 JP 2004268306
(71) Applicant: Takeda Pharmaceutical Company Limited, Osaka-shi, Osaka 541-0045 (JP)
(72) Inventor: INOUE, Kinji, Saitama-shi, Saitama 3380825 (JP); USUI, Kimie, Tsukuba-shi, Ibaraki 3004293 (JP)
(74) Representative: Kalhammer, Georg
(86) International application number: PCT/JP2005/017324
(87) International publication number: WO 2006/030956

(57) **Abstract**

The present invention relates to provision of a method of screening a medicament using a disease-associated gene product, an antibody to the disease-associated gene product, and an antisense DNA which suppresses expression of the disease-associated gene product. Specifically, the present invention provides a protein comprising the same or substantially the same amino acid sequence as the amino acid sequence represented by SEQ ID NO: 1, its partial peptide, or a salt thereof.

## Description

### TECHNICAL FIELD

The present invention relates to a method of screening a medicament for the prevention/treatment/improvement of Parkinson's disease, as a novel use of known PrRP and its receptors, and so on.

### BACKGROUND ART

Human-, bovine- and rat-derived ligand polypeptides (PrRP) having a pituitary function modulating activity, a central nervous system modulating activity and a pancreatic function regulating activity and cDNA encoding the same, as well as human- and mouse-derived receptor proteins to said PrRP (PrRP receptors) and cDNA encoding the same are known (WO 97/24436).

Mouse-derived PrRP and genomic DNA encoding the same, and a method of producing PrRP knockout mouse and a targeting vector therefor are known (WO 98/49295).

Rat-derived orphan receptor (UHR-1) with high homology to human- and mouse-derived PrRP receptors are known (Biochemical and Biophysical Research Communications, Vol. 209, No. 2, pp. 606-613, 1995).

PrRP is known to have a prolactin secretion modulating activity and a placental function modulating activity (WO 98/58962).

PrRP is known to have an oxytocin secretion modulating activity (WO 00/38704).

Monoclonal antibodies (P2L-1Ca P2L-2Ca, P2L-1Ta) to PrRP are known (WO 99/60112).

It is known that PrRP has an adrenocorticotropic hormone release secretion modulating activity and is effective for the prevention/treatment of obesity (WO. 00/38704).

PrRP knockout animals that develop obesity, etc. are known (JPA 2004-121243).

It is described that PrRP is associated with eating regulation (Nature Neuroscience Vol. 3, No. 7, July 2000).

It is known that eating and body weight gain are inhibited by administration of PrRP (Endocrinology February 2002, 143(2): 360-367)

It is known that eating and body weight gain are inhibited by interaction of PrRP and leptin (Endocrinology February 2002, 143(2): 368-374).

### DISCLOSURE OF THE INVENTION

The present invention aims at elucidating additional functions of PrRP and PrRP receptors and providing novel medicaments.

In order to solve the problems above, the present inventors made extensive investigations and found that Parkinson's disease is not developed in mice deficient in PrRP gene expression (PrRP knockout mice), suggesting that PrRP could promote the onset of Parkinson's disease and PrRP antagonists could be medicaments for the treatment of Parkinson's disease. The present inventors made further investigations and as a result, also found that Parkinson's disease induced by administration of MPTP (Parkinson's disease inducer) is not developed even by administration of PrRP neutralizing antibody. Thus, the present invention has come to be accomplished.

That is, the present invention provides the following features:
(1) a method of screening a substance for the prevention/treatment of a disease associated with the function of a PrRP receptor (e.g., a disease selected from the group consisting of Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism, hypertension, burn, psychosomatic disorders and schizophrenia), which comprises using a protein comprising the same or substantially the same amino acid sequence as the PrRP receptor and/or a protein comprising the same or substantially the same amino acid sequence as PrRP, or a salt thereof;
(2) a method of screening a substance for the prevention/treatment of a disease associated with the function of a PrRP receptor, which comprises using the PrRP receptor and/or PrRP, or a salt thereof;
(3) a method of screening a substance for the prevention/treatment of a disease associated with the function of a PrRP receptor, which comprises using the PrRP receptor or a salt thereof;
(4) the screening method according to any one of (1) to (3), wherein the disease is Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism or psychosomatic disorders;
(5) the screening method according to any one of (1) to (3), wherein the disease is Parkinson's disease;
(6) the screening method according to any one of (1) to (3), wherein the disease is schizophrenia;
(7) a method of preventing/treating a disease associated with the function of a PrRP receptor, which comprises inhibiting the binding of PrRP receptor and PrRP;
(8) the preventing/treating method according to (7), wherein the disease is Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism or psychosomatic disorders (preferably, Parkinson's disease, motor function disorders, depression, neurosis, psychosomatic disorders, etc.; more preferably Parkinson's disease, etc.);
(9) the preventing/treating method according to (7), wherein the disease is Parkinson's disease;
(10) the preventing/treating method according to any one of (7) to (9), wherein an antagonist to the PrRP receptor is used;
(11) the preventing/treating method according to any one of (7) to (9), wherein an antibody to the PrRP receptor is used;
(12) the preventing/treating method according to any one of (7) to (9), wherein an antibody to PrRP (preferably a neutralizing antibody)is used;
(13) a method of preventing/treating a disease associated with the function of a PrRP receptor, which comprises inhibiting the expression of a PrRP receptor and/or PrRP;
(14) the preventing/treating method according to (13), wherein the disease is Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism or psychosomatic disorders;
(15) the preventing/treating method according to (13), wherein the disease is Parkinson's disease;
(16) the preventing/treating method according to any one of (13) to (15), wherein an antisense polynucleotide inhibiting the expression of PrRP receptor and/or PrRP is used;
(17) the preventing/treating method according to any one of (13) to (15), wherein a siRNA inhibiting the expression of PrRP receptor and/or PrRP is used;
(18) an agent for the prevention/treatment of a disease associated with the function of a PrRP receptor, which comprises a substance inhibiting the binding of a PrRP receptor and PrRP;
(19) the agent for the prevention/treatment according to (18), wherein the disease is Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism or psychosomatic disorders;
(20) the agent for the prevention/treatment according to (18), wherein the disease is Parkinson's disease;
(21) the agent for the prevention/treatment according to any one of (18) to (21), wherein the substance inhibiting the binding of PrRP receptor and PrRP is an antagonist to the PrRP receptor;
(22) the agent for the prevention/treatment according to any one of (18) to (21), wherein the substance inhibiting the binding of PrRP receptor and PrRP is an antibody to the PrRP receptor;
(23) the agent for the prevention/treatment according to any one of (18) to (21), wherein the substance inhibiting the binding of PrRP receptor and PrRP is an antibody to PrRP (preferably a neutralizing antibody);
(24) an agent for the prevention/treatment of a disease associated with the function of a PrRP receptor, which comprises a substance inhibiting the expression of PrRP receptor and/or PrRP;
(25) the agent for the prevention/treatment according to (24), wherein the disease is Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism or psychosomatic disorders;
(26) the agent for the prevention/treatment according to (24), wherein the disease is Parkinson's disease;
(27) the agent for the prevention/treatment according to any one of (24) to (26), wherein the substance inhibiting the expression of PrRP receptor and/or PrRP is an antisense polynucleotide;
(28) the agent for the prevention/treatment according to any one of (24) to (26), wherein the substance inhibiting the expression of PrRP receptor and/or PrRP is a siRNA;
(29) a method of preventing/treating a disease associated with the function of a PrRP receptor, which comprises promoting the binding of the PrRP receptor and PrRP;
(30) the preventing/treating method according to (29), wherein the disease is schizophrenia;
   (30a) a method of preventing/treating a disease associated with the function of a PrRP receptor, which comprises promoting the function of a PrRP receptor (herein "promoting the function of a PrRP receptor" has the same significance as "promoting the activity of a PrRP receptor," "enhancing the function of a PrRP receptor" or "enhancing the activity of a PrRP receptor");
   (30b) the preventing/treating method according to (30a), wherein the disease is schizophrenia;
   (30c) the preventing/treating method according to (30a) or (30b), wherein an agonist to the PrRP receptor is used;
(31) an agent for the prevention/treatment of a disease associated with the function of a PrRP receptor, which comprises a substance promoting the function of PrRP receptor;
(32) the agent for the prevention/treatment according to (31), wherein the disease is schizophrenia;
(33) the agent for the prevention/treatment according to (31) or (32), wherein the substance promoting the function of PrRP receptor is an agonist for the PrRP receptor;
(34) a method of diagnosis for a disease associated with the function of a PrRP receptor, which comprises assaying the expression level of a PrRP receptor gene and/or a PrRP gene, or an increase or decrease of the level of PrRP receptor and/or PrRP produced;
   (34a) a method of diagnosis for a disease associated with the function of a PrRP receptor, which comprises assaying the expression level of a PrRP receptor gene and/or a PrRP gene, or an increase of the level of PrRP receptor and/or PrRP produced;
   (34b) a method of diagnosis for a disease associated with the function of a PrRP receptor, which comprises assaying the expression level of a PrRP receptor gene and/or a PrRP gene, or a decrease of the level of PrRP receptor and/or PrRP produced;
(35) the method for diagnosis according to (34a), wherein the disease is Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism, hypertension or psychosomatic disorders;
(36) the method for diagnosis according to (34), wherein the disease is Parkinson's disease;
(37) the method for diagnosis according to (34b), wherein the disease is schizophrenia;
(38) a kit for diagnosis of a disease associated with the function of a PrRP receptor, which comprises (1) an antibody to PrRP, an antibody to PrRP receptor, a PrRP gene or a PrRP receptor gene, and (2) a buffer;
(39) the kit for diagnosis according to (38), wherein the disease is Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism, hypertension or psychosomatic disorders;
(40) the kit for diagnosis according to (38), wherein the disease is Parkinson's disease;
(41) the kit for diagnosis according to (38), wherein the disease is schizophrenia; and so on.

The present invention inhibits the function of PrRP and suppresses the expression of PrRP and is thus useful as a medicament for the prevention/treatment/improvement of Parkinson's disease.

### BRIEF DESCRIPTION OF THE DRAWINGS

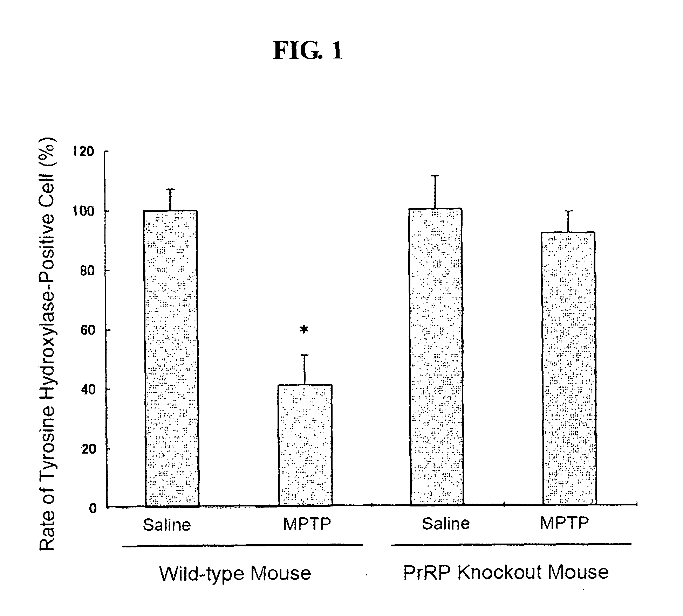

FIG. 1 shows rates of nigrostriatal tyrosine hydroxylase-positive cells in the PrRP knockout mice and wild-type mice administered with saline or MPTP. The values are mean ± SE (n=5). Symbol * denotes p<0.05 vs. the wild-type mice administered with saline (Student's t-test).

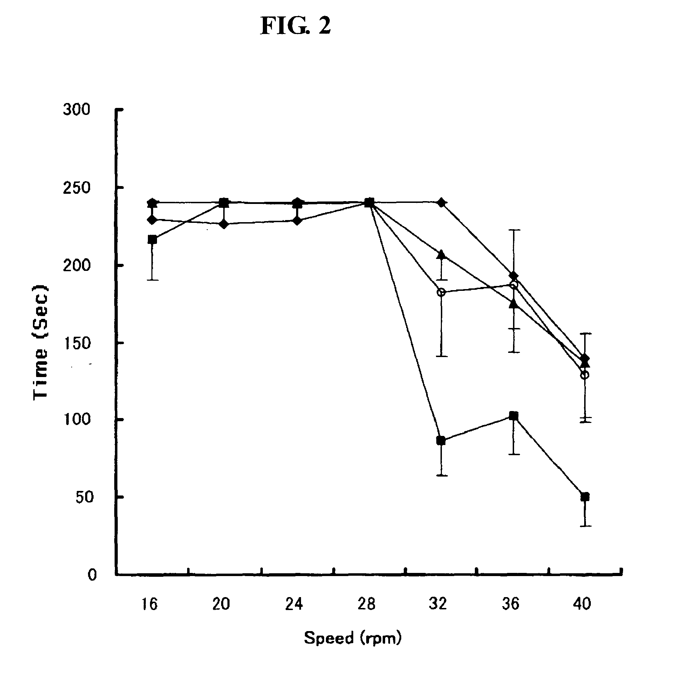

FIG. 2 shows the measurement values of motor activities using a rotarod in the PrRP knockout mice and wild-type mice administered with saline or MPTP. The values are mean ± SE (n=5). Symbol ◆(closed diamond) denotes changes in motor activities of the wild-type mice administered with saline; symbol ■(closed square), the wild-type mice administered with MPTP; symbol ▲(closed triangle), the PrRP knockout mice administered with saline; and, symbol O(open circle), the PrRP knockout mice administered with MPTP; respectively.

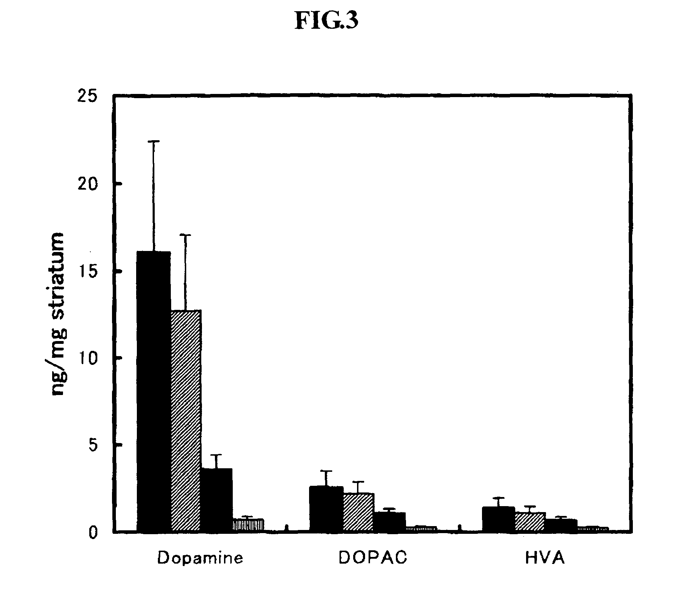

FIG. 3 shows the measurement values of catecholamines in the striata of PrRP knockout mice and wild-type mice when saline or MPTP was administered. The values are mean ± SE (n=5). The black bar denotes catecholamine levels in the striatum of the wild-type mice administered with saline; the slant-shaded bar, the PrRP knockout mice administered with saline; the gray bar, the wild-type mice administered with MPTP; and the striped bar, the PrRP knockout mice administered with MPTP.

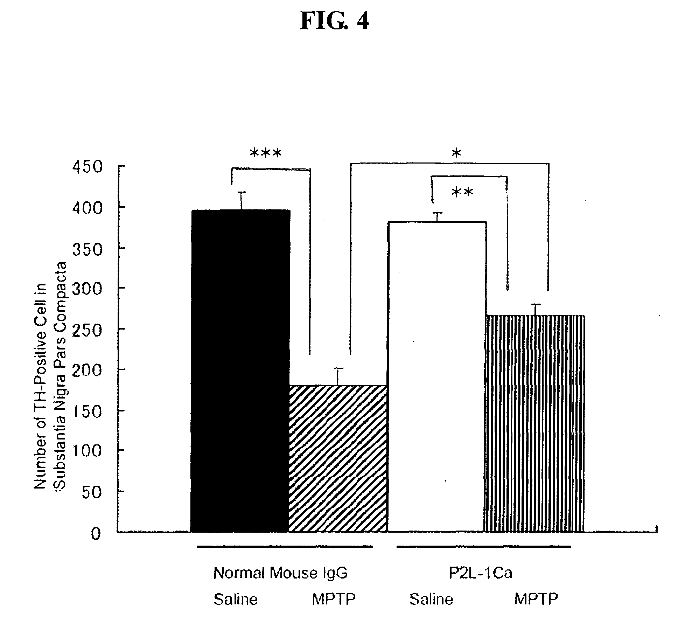

FIG. 4 shows the number of TH-positive cells in the substantia nigra pars compacta when MPTP was administered after administration of anti-PrRP neutralizing antibody and normal mouse IgG. The values are mean ± SE (n=3). The black bar denotes the number of TH-positive cells in the substantia nigra of the mice administered with saline after administration of normal mouse IgG; the slant-shaded bar, the mice administered with MPTP after administration of normal mouse IgG; the gray bar, the mice administered with saline after administration of anti-PrRP neutralizing antibody (P2L-1Ca); and the striped bar, the mice administered with MPTP after administration of anti-PrRP neutralizing antibody (P2L-1Ca).
   Symbol * denotes p<0.05, the mice administered with MPTP after administration of normal mouse IgG vs. the mice administered with MPTP after administration of anti-PrRP neutralizing antibody (P2L-1Ca).
   Symbol ** denotes p<0.01, the mice administered with saline after administration of anti-PrRP neutralizing antibody (P2L-1Ca) vs. the mice administered with MPTP after administration of anti-PrRP neutralizing antibody (P2L-1Ca).
   Symbol *** denotes p<0.001, the mice administered with saline after administration of normal mouse IgG vs. the mice administered with MPTP after administration of normal mouse IgG.

   The assay was carried out using the Tukey's test.

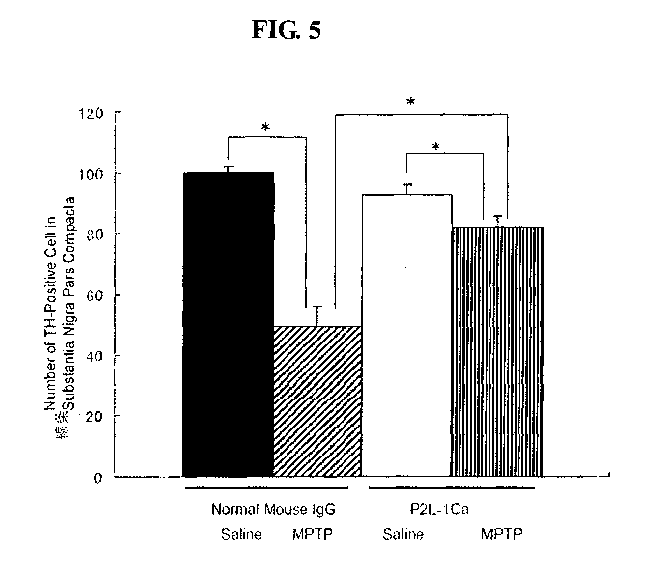

FIG. 5 shows TH nerve fiber density in the striatum when MPTP was administered after administration of anti-PrRP neutralizing antibody and normal mouse IgG. The black bar denotes TH-positive nerve cell density of the mice administered with saline after administration of normal mouse IgG; the slant-shaded bar, the mice administered with MPTP after administration of normal mouse IgG; the gray bar, the mice administered with saline after administration of anti-PrRP neutralizing antibody (P2L-1Ca); and the striped bar, the mice administered with saline after administration of anti-PrRP neutralizing antibody (P2L-1 Ca). The TH-positive nerve density of the mice administered with saline after administration of normal mouse IgG was expressed 100% and the values denote mean ± SE (n=3).
   Symbol * denotes p<0.05; the tests for the mice administered with MPTP after administration of normal mouse IgG vs. the mice administered with MPTP after administration of anti-PrRP neutralizing antibody (P2L-1Ca); the mice administered with saline after administration of normal mouse IgG vs. the mice administered with MPTP after administration of normal mouse IgG; and the mice administered with saline after administration of normal mouse IgG vs. the mice administered with MPTP after administration of anti-PrRP neutralizing antibody (P2L-1Ca) were performed using the Steel-Dwass test.

### BEST MODE FOR CARRYING OUT THE INVENTION

Throughout the specification, PrRP refers to the protein comprising the same or substantially the same amino acid sequence as the amino acid sequence represented by SEQ ID NO: 1, preferably the protein comprising the amino acid sequence represented by SEQ ID NO: 1.

In the present invention, the protein comprising the same or substantially the same amino acid sequence as the amino acid sequence represented by SEQ ID NO: 1 (hereinafter referred to as the protein of the present invention) may be any protein derived from any cells of mammal (e.g., human, mouse, rat, rabbit, sheep, swine, bovine, horse, fowl, cat, dog, monkey, chimpanzee, etc.) (e.g., hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, prostate, ovary, placenta, uterus, bone, joint, skeletal muscle, etc.; the proteins may also be synthetic proteins.

The amino acid sequence which has the same or substantially the same as that represented by SEQ ID NO: 1 includes an amino acid sequence having at least about 50% homology, preferably at least about 60% homology, more preferably at least about 70% homology, much more preferably at least about 80% homology, most preferably at least about 90% homology, to the amino acid sequence represented by SEQ ID NO: 1; and the like.

The protein comprising substantially the same amino acid sequence as the amino acid sequence represented by SEQ ID NO: 1 preferably includes, for example, a protein comprising substantially the same amino acid sequence as the amino acid sequence represented by SEQ ID NO: 1 described above and having substantially the same activity as the protein comprising the amino acid sequence represented by SEQ ID NO: 1, and the like.

Examples of substantially the same activity include an activity of releasing prolactin from pituitary primary culture cells, an activity of suppressing food intake, an activity of promoting adrenocorticotropic hormone secretion, an activity of promoting oxytocin secretion, an activity of promoting vasopressin secretion, an activity of raising blood pressure, and the like. The term "substantially the same" is used to mean that the natures of their activities are equivalent to one another (e.g., physiologically or pharmacologically). It is thus preferred that though the transcriptional regulatory activity be equivalent (e.g., approximately 0.01 to 100 times, preferably about 0.1 to 10 times, more preferably about 0.5 to 2 times), quantitative factors such as degrees of these activities and molecular weight of the proteins may differ from each other.

Examples of the PrRP used in the present invention include so-called muteins such as proteins comprising (1) (i) the amino acid sequence represented by SEQ ID NO: 1, of which at least 1 or 2 (e.g., about 1 to about 30, preferably about 1 to about 10, more preferably several (1 to 5)) amino acids are deleted; (ii) the amino acid sequence represented by SEQ ID NO: 1, to which at least 1 or 2 (e.g., about 1 to about 30, preferably about 1 to about 10, more preferably several (1 to 5)) amino acids are added; (iii) the amino acid sequence represented by SEQ ID NO: 1, in which at least 1 or 2 (e.g., about 1 to about 30, preferably about 1 to about 10, more preferably several (1 to 5)) amino acids are inserted; (iv) the amino acid sequence represented by SEQ ID NO: 1, in which at least 1 or 2 (e.g., about 1 to about 30, preferably about 1 to about 10, more preferably several (1 to 5)) amino acids are substituted by other amino acids; or (v) a combination of these amino acid sequences; and the like.

Where the amino acid sequence is inserted, deleted or substituted as described above, the position of its insertion, deletion or substitution is not particularly limited.

Throughout the specification, the PrRP receptor is used to mean the protein comprising the same or substantially the same amino acid sequence as the amino acid sequence represented by SEQ ID NO: 9, preferably the protein comprising the amino acid sequence represented by SEQ ID NO: 9.

In the present invention, the protein comprising the same or substantially the same amino acid sequence as the amino acid sequence represented by SEQ ID NO: 9 (hereinafter referred to as the protein of the present invention) may be any protein derived from any cells of mammal (e.g., human, mouse, rat, rabbit, sheep, swine, bovine, horse, fowl, cat, dog, monkey, chimpanzee, etc.) (e.g., hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, prostate, ovary, placenta, uterus, bone, joint, skeletal muscle, etc.; the proteins may also be synthetic proteins.

The amino acid sequence which has the same or substantially the same as that represented by SEQ ID NO: 9 includes an amino acid sequence having at least about 50% homology, preferably at least about 60% homology, more preferably at least about 70% homology, much more preferably at least about 80% homology, most preferably at least about 90% homology, to the amino acid sequence represented by SEQ ID NO: 9.

The protein comprising the same or substantially the same amino acid sequence as the amino acid sequence represented by SEQ ID NO: 9 includes, e.g., a protein comprising substantially the same amino acid sequence as the amino acid sequence represented by SEQ ID NO: 9 described above and having substantially the same activity as the protein comprising the amino acid sequence represented by SEQ ID NO: 9.

Examples of substantially the same activity include an activity of releasing prolactin from pituitary primary culture cells, an activity of suppressing food intake, an activity of promoting adrenocorticotropic hormone secretion, an activity of promoting oxytocin secretion, an activity of promoting vasopressin secretion, an activity of raising blood pressure, and the like. The term "substantially the same" is used to mean that the natures of their activities are equivalent to one another (e.g., physiologically or pharmacologically). It is thus preferred that though the transcriptional regulatory activity be equivalent (e.g., approximately 0.01 to 100 times, preferably about 0.1 to 10 times, more preferably about 0.5 to twice), quantitative factors such as degrees of these activities and molecular weight of the proteins may differ from each other.

Examples of the PrRP receptor used in the present invention include so-called muteins such as proteins comprising (1) (i) the amino acid sequence represented by SEQ ID NO: 9, of which at least 1 or 2 (e.g., about 1 to about 30, preferably about 1 to about 10, more preferably several (1 to 5)) amino acids are deleted; (ii) the amino acid sequence represented by SEQ ID NO: 9, to which at least 1 or 2 (e.g., about 1 to about 30, preferably about 1 to about 10, more preferably several (1 to 5)) amino acids are added; (iii) the amino acid sequence represented by SEQ ID NO: 9, in which at least 1 or 2 (e.g., about 1 to about 30, preferably about 1 to about 10, more preferably several (1 to 5)) amino acids are inserted; (iv) the amino acid sequence represented by SEQ ID NO: 9, in which at least 1 or 2 (e.g., about 1 to about 30, preferably about 1 to about 10, more preferably several (1 to 5)) amino acids are substituted by other amino acids; or (v) a combination of these amino acid sequences; and the like.

Where the amino acid sequence is inserted, deleted or substituted as described above, the position of its insertion, deletion or substitution is not particularly limited.

Throughout the present specification, the proteins are represented in accordance with the conventional way of describing peptides, that is, the N-terminus (amino terminus) at the left hand and the C-terminus (carboxyl terminus) at the right hand. In the proteins of the present invention including the proteins containing the amino acid sequence shown by SEQ ID NO: 1, the C-terminus is usually in the form of a carboxyl group (-COOH) or a carboxylate (-COO⁻) but may be in the form of an amide (-CONH₂) or an ester (-COOR).

Examples of the ester group shown by R include an alkyl group having 1 to 6 carbon atoms such as methyl, ethyl, n-propyl, isopropyl, n-butyl, etc.; a cycloalkyl group having 3 to 8 carbon atoms such as cyclopentyl, cyclohexyl, etc.; an aryl group having 6 to 12 carbon atoms such as phenyl, α-naphthyl, etc.; an aralkyl having 7 to 14 carbon atoms such as a phenyl-C₁₋₂ alkyl group, e.g., benzyl, phenethyl, etc.; an α-naphthyl-C₁₋₂ alkyl group, e.g., α-naphthylmethyl, etc.; and the like. In addition, pivaloyloxymethyl or the like which is used widely as an ester for oral administration may also be used.

Where the protein of the present invention contains a carboxyl group (or carboxylate) at a position other than the C-terminal, it may be amidated or esterified and such an amide or ester is also included within the protein of the present invention. The ester group used in this case includes, for example, the C-terminal ester, etc. described above.

Further, the protein of the present invention includes derivatives wherein the amino group of N-terminal amino acid residues (e.g., methionine residue) of the above protein is protected with a protecting group (e.g., a C₁₋₆ acyl group such as a C₁₋₆ alkanoyl, etc., including formyl group, acetyl group, etc.); those wherein the N-terminal region is cleaved in vivo and the glutamyl group formed is pyroglutaminated; and those wherein a substituent (e.g., -OH, -SH, amino group, imidazole group, indole group, guanidino group, etc.) on the side chains of an amino acid in the molecule of the protein is protected with an appropriate protecting group (e.g., a C₁₋₆ acyl group such as a C₁₋₆ alkanoyl, etc., including formyl group, acetyl group, etc.), or conjugated proteins such as glycoproteins with sugar chains attached thereto.

As the salts of the protein of the present invention, salts with physiologically acceptable acids (e.g., inorganic acids, organic acids) or with bases (e.g., alkaline metal salts) are employed, and physiologically acceptable acid addition salts are particularly preferred. Examples of such salts are salts with inorganic acids (e.g., hydrochloric acid, phosphoric acid, hydrobromic acid or sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Where the proteins of the present invention or salt thereof can be produced from the mammalian tissues or cells described above by publicly known methods for purification of proteins. Specifically, the proteins of the present invention or salt thereof can be produced by homogenizing these mammalian tissues or cells and separating/purifying the soluble fractions by chromatography techniques such as reversed phase chromatography, ion exchange chromatography, and the like.

The protein of the present invention or its salt may also be manufactured in accordance with peptide synthesis methods publicly known.

For the methods of peptide synthesis, for example, either solid phase synthesis or liquid phase synthesis may be used. The partial peptide or amino acids that can construct the protein of the present invention are condensed with the remaining part. Where the product contains protecting groups, these protecting groups are removed to give the desired protein.

Herein, condensation and elimination of the protecting groups are performed by publicly known methods described in (1)-(5) below.
1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: Peptide Gosei-no-Kiso to Jikken (Basics and experiments of peptide synthesis), published by Maruzen Co. (1975)
4) Haruaki Yajima & Shunpei Sakakibara: Seikagaku Jikken Koza (Biochemical Experiment) 1, Tanpakushitsu no Kagaku (Chemistry of Proteins) IV, 205 (1977)
5) Haruaki Yajima ed.: Zoku lyakuhin no Kaihatsu (A sequel to Development of Pharmaceuticals), Vol. 14, Peptide Synthesis, published by Hirokawa Shoten

The protein thus obtained may be purified and isolated by conventional purification methods. Herein, the purification methods include solvent extraction, distillation, column chromatography, liquid chromatography, recrystallization and a combination thereof.

When the protein obtained by the above methods is in a free form, the protein can be converted into an appropriate salt by a publicly known method or its modification; when the protein is obtained in a salt form, it can be converted into a free form or other different salt form by a publicly known method or its modification.

To synthesize the protein of the present invention or its salts, commercially available resins that are used for protein synthesis can be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenyl-hydroxymethyl)phenoxy resin and 4-(2',4'-dimethoxyphenyl-Fmoc-aminoethyl) phenoxy resin.

Using these resins, amino acids in which α-amino groups and functional groups on the side chains are appropriately protected are condensed on the resin in the order of the sequence of the objective protein according to various condensation methods publicly known in the art. At the end of the reaction, the protein is excised from the resin and at the same time, the protecting groups are removed. Then, intramolecular disulfide bond-forming reaction is performed in a highly diluted solution to obtain the objective protein or amides thereof.

For condensation of the protected amino acids described above, a variety of activation reagents for protein synthesis may be used, but carbodiimides are particularly preferably employed. Examples of such carbodiimides include DCC, N,N'-diisopropylcarbodiimide, N-ethyl-N'-(3-dimethylaminopropyl)carbodiimide, etc.

For condensation of the protected amino acids, the protected amino acids are added directly to the resin together with a racemization inhibitor (e.g., HOBt, HOOBt), or the protected amino acids are previously activated in the form of symmetric acid anhydrides, HOBt esters or HOOBt esters, followed by adding the thus activated protected amino acids to the resin.

Solvents suitable for use in the activation of the protected amino acids or the condensation with the resin may be selected from solvents that are known to be usable for protein condensation reactions. Examples of such solvents are acid amides such as N,N-dimethylformamide, N,N-dimethylacetamide and N-methylpyrrolidone; halogenated hydrocarbons such as methylene chloride and chloroform; alcohols such as trifluoroethanol; sulfoxides such as dimethylsulfoxide; ethers such as pyridine, dioxane and tetrahydrofuran; nitriles such as acetonitrile and propionitrile; esters such as methyl acetate and ethyl acetate; and appropriate mixtures of these solvents.

The reaction temperature is appropriately chosen from the range known to be applicable to protein condensation reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

The carboxyl group in the starting amino acids can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl and 2-adamantyl), aralkyl esterification (e.g., benzyl ester, 4-nitrobenzyl ester, 4-methoxybenzyl ester, 4-chlorobenzyl ester and benzhydryl ester), phenacyl esterification, benzyloxycarbonyl hydrazidation, t-butoxycarbonyl hydrazidation, trityl hydrazidation, etc.

The hydroxyl group of serine can be protected through, for example, its esterification or etherification. Examples of groups appropriately used for the esterification include a lower (C₁₋₆) alkanoyl group such as acetyl group, an aroyl group such as benzoyl group, and a group derived from carbonic acid such as benzyloxycarbonyl group and ethoxycarbonyl group. Examples of a group appropriately used for the etherification include benzyl group, tetrahydropyranyl group and t-butyl group.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting amino acids include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the activated amino acids in which the amino groups in the starting amino acids are activated, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination reaction of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

The amidated protein can be prepared by amidation of the α-carboxyl group of the carboxy terminal amino acid of the protein in accordance with publicly known methods.

The esterified protein can be prepared, for example, by condensation of the α-carboxyl group of the carboxy terminal amino acid of the protein with a desired alcohol.

In addition, the protein of the present invention or its salt can also be manufactured by culturing a transformant bearing the DNA encoding the protein of the present invention, and separating and purifying the protein of the present invention or its salt from the resulting culture.

The DNA encoding the protein of the present invention may also be any one of genomic DNA, genomic DNA library, cDNA derived from the cells or tissues described above, cDNA library derived from the cells or tissues described above and synthetic DNA. The vector to be used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

The DNA encoding the protein of the present invention (PrRP) may be any one of, for example, DNA comprising the base sequence represented by SEQ ID NO: 2, SEQ ID NO: 4, SEQ ID NO: 6 or SEQ ID NO: 8, or any DNA comprising a base sequence hybridizable to the base sequence represented by SEQ ID NO: 2, SEQ ID NO: 4, SEQ ID NO: 6 or SEQ ID NO: 8 under high stringent conditions and encoding a protein which has the activities substantially equivalent to those of the protein comprising the amino acid sequence represented by SEQ ID NO: 1 described above (e.g., a ligand binding activity, a signal transduction activity, etc.). Hereinafter, when the protein of the present invention refers to the PrRP receptor, "SEQ ID NO: 2, SEQ ID NO: 4, SEQ ID NO: 6 or SEQ ID NO: 8" is deemed to be replaced with "SEQ ID NO: 10."

Examples of the DNA that is hybridizable to the base sequence represented by SEQ ID NO: 2, SEQ ID NO: 4, SEQ ID NO: 6 or SEQ ID NO: 8 include DNA having at least about 50% homology, preferably at least about 60% homology, more preferably at least about 70% homology, much more preferably at least about 80% homology, still much more preferably at least about 90% homology and most preferably at least about 95% homology, to the amino acid sequence represented by SEQ ID NO: 2, SEQ ID NO: 4, SEQ ID NO: 6 or SEQ ID NO: 8; and the like.

The hybridization can be carried out by publicly known methods or by a modification thereof, for example, according to the method described in Molecular Cloning, 2nd Ed.; J. Sambrook et al., Cold Spring Harbor Lab. Press, (1989). A commercially available library may also be used according to the instructions of the attached manufacturer's protocol. The hybridization can be carried out preferably under high stringent conditions.

The high stringent conditions used herein are, for example, those in a sodium concentration at about 19 mM to about 40 mM, preferably about 19 mM to about 20 mM and a temperature at about 50°C to about 70°C, preferably about 60°C to about 65°C. In particular, hybridization conditions in a sodium concentration at about 19 mM and a temperature at about 65°C are most preferred.

Preferably, the DNA encoding the protein comprising the amino acid sequence represented by SEQ ID NO: 1 includes DNA comprising the base sequence represented by SEQ ID NO: 2, SEQ ID NO: 4, SEQ ID NO: 6 or SEQ ID NO: 8.

For cloning of the DNA that completely encodes the protein of the present invention, the DNA may be either amplified by PCR using synthetic DNA primers containing a part of the base sequence of the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). The hybridization may also be performed using commercially available library in accordance with the protocol described in the attached instructions.

The base sequence of DNA can be substituted by publicly known methods such as the ODA-LA PCR method, the Gapped duplex method, the Kunkel method, etc., or its modification, using a publicly known kit available as Mutan^{™}-super Express Km (manufactured by Takara Shuzo Co., Ltd.) or Mutan^{™}-K (manufactured by Takara Shuzo Co., Ltd.), etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector described above can be manufactured, for example, by excising the desired DNA fragment from the DNA encoding the protein of the present invention, and then ligating the DNA fragment with an appropriate expression vector downstream a promoter in the vector.

Examples of the vector which can be used include plasmids derived form E. coli (e.g., pBR322, pBR325, pUC12, pUC13), plasmids derived from *Bacillus subtilis* (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter may be any promoter if it matches well with a host to be used for gene expression.

In the case of using animal cells as the host, examples of the promoter include SRα promoter, SV40 promoter, LTR promoter, CMV promoter, HSV-TK promoter, etc. Among them, CMV promoter, SRα promoter, etc. are preferably used.

Where the host is bacteria of the genus Escherichia, preferred examples of the promoter include trp promoter, lac promoter, recA promoter, λP_{L} promoter, lpp promoter, etc.

In the case of using bacteria of the genus Bacillus as the host, preferred example of the promoter are SPO1 promoter, SPO2 promoter, penP promoter, and the like.

In the case of using yeast as the host, preferred examples of the promoter are PHO5 promoter, PGK promoter, GAP promoter, ADH promoter, and the like.

In the case of using insect cells as the host, preferred examples of the promoter include polyhedrin promoter, P10 promoter, and the like.

In addition to the foregoing examples, the expression vector may further optionally contain an enhancer, a splicing signal, a poly A addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo, G418 resistance), etc. In particular, when CHO (dhfr⁻) cell is used together with dhfr gene as the selection marker, selection can also be made by using a thymidine free medium.

If necessary, a signal sequence that matches with a host is added to the N-terminus of the protein of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

The transformant bearing the DNA that encodes the protein of the present invention can be produced by transformation of a host with an expression vector bearing the above DNA in accordance with publicly known methods.

Herein, the expression vector includes the vector described above.

Examples of the host, which may be employed, are bacteria belonging to the genus Escherichia, bacteria belonging to the genus Bacillus, yeast, insect cells, insects and animal cells, etc.

Specific examples of bacteria belonging to the genus Escherichia include *Escherichia coli* K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of bacteria belonging to the genus Bacillus include *Bacillus subtilis* MI114 [Gene, 24, 255 (1983)], 207-21 [Journal of Biochemistry, 95, 87 (1984)], etc.

Examples of yeast include *Saccharomyces cereviseae* AH22, AH22R-, NA87-11A, DKD-5D, 20B-12, *Schizosaccharomyces pombe* NCYC1913, NCYC2036, Pichia pastoris KM71, etc.

Examples of insect cells include, for the virus AcNPV, *Spodoptera frugiperda* cell (Sf cell), MG1 cell derived from mid-intestine of *Trichoplusia ni,* High Five^{™} cell derived from egg of Trichoplusia ni, cells derived from *Mamestra brassicae,* cells derived from *Estigmena acrea,* etc.; and for the virus BmNPV, *Bombyx mori* N cell (BmN cell), etc. Examples of the Sf cell which can be used are Sf9 cell (ATCC CRL1711) and Sf21 cell (both cells are described in Vaughn, J. L. et al., In Vivo, 13, 213-217 (1977).

As the insect, for example, a larva of *Bombyx mori* can be used [Maeda et al., Nature, 315, 592 (1985)].

Examples of animal cells include simian cell COS-7, Vero, Chinese hamster cell CHO (hereinafter referred to as CHO cell), dhfr gene deficient Chinese hamster cell CHO (hereinafter simply referred to as CHO(dhfr⁻) cell), mouse L cell, mouse AtT-20, mouse myeloma cell, rat GH 3, human FL cell, etc.

Transformation can be performed in accordance with publicly known methods, depending on the type of the host.

Bacteria belonging to the genus Escherichia can be transformed, for example, according to the method described in Proc. Natl. Acad. Sci. U.S.A., 69, 2110 (1972), Gene, 17, 107 (1982), etc.

Bacteria belonging to the genus Bacillus can be transformed, for example, according to the method described in Molecular & General Genetics, 168, 111 (1979), etc.

Yeast can be transformed, for example, according to the method described in Methods in Enzymology, 194, 182-187 (1991) or Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995), published by Shujunsha, or Virology, 52, 456 (1973).

Incubation of the transformant can be performed in accordance with publicly known methods, depending on the type of the host.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary, a chemical such as 3β-indolylacrylic acid can be added to the medium thereby to activate the promoter efficiently.

Where the bacteria belonging to the genus Escherichia are used as the host, the transformant is usually cultivated at about 15°C to about 43°C for about 3 hours to about 24 hours. If necessary, the culture may be aerated or agitated.

Where the bacteria belonging to the genus Bacillus are used as the host, the transformant is cultivated generally at about 30°C to about 40°C for about 6 hours to about 24 hours. If necessary, the culture can be aerated or agitated.

Where yeast is used as the host, the transformant is cultivated in, for example, Burkholder's minimal medium [Bostian, K. L. et al., Proc. Natl. Acad. Sci. U.S.A., 77, 4505 (1980)], in SD medium supplemented with 0.5% Casamino acids [Bitter, G. A. et al., Proc. Natl. Acad. Sci. U.S.A., 81, 5330 (1984)], etc. Preferably, pH of the medium is adjusted to about 5 to about 8. In general, the transformant is cultivated at about 20°C to about 35°C for about 24 hours to about 72 hours. If necessary, the culture can be aerated or agitated.

Where insect cells or insects are used as the host, the transformant is cultivated in, for example, Grace's Insect Medium [Grace, T.C.C., Nature, 195, 788 (1962)] to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultivated in, for example, MEM medium containing about 5% to about 20% fetal bovine serum (Science, 122, 501 (1952)], DMEM medium [Virology, 8, 396 (1959)), RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours and, if necessary, the culture can be aerated or agitated.

As described above, the protein of the present invention can be produced in the cell or cell membrane or outside the cell, of the transformant.

From the culture obtained by culturing the above transformant, the protein of the present invention can be separated and purified in accordance with publicly known methods.

For example, when the protein of the present invention is extracted from the cultured cells or cells, the cultured cell or cell is collected by a publicly known method and suspended in an appropriate buffer. The cultured cell or cell is then disrupted by a publicly known method such as ultrasonication, a treatment with lysozyme and/or freeze-thaw cycling, followed by centrifugation, filtration, etc. Thus, the crude extract of the protein can be obtained. The buffer used for the procedures may contain a protein modifier such as urea or guanidine hydrochloride, or a surfactant such as Triton X-100^{™}, etc. When the protein is secreted in the culture, the culture supernatant can be collected from the culture by a publicly known method.

The culture supernatant or the protein contained in the extract thus obtained can be separated and purified by publicly known methods. Such methods include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like. These methods can also be combined appropriately.

When the protein thus obtained is in a free form, it can be converted into its salt by publicly known methods or modifications thereof. On the other hand, when the protein is obtained in the form of a salt, it can be converted into the free form or in the form of a different salt by publicly known methods or modifications thereof.

The protein produced by the transformant can be treated, prior to or after the purification, with an appropriate protein modifying enzyme so that the protein can be appropriately modified to partially remove a polypeptide. Examples of the protein-modifying enzyme include trypsin, chymotrypsin, arginyl endopeptidase, protein kinase, glycosidase and the like.

The presence of the protein of the present invention thus obtained can be determined by an enzyme immunoassay using a specific antibody, western blotting, or the like.

The disease associated with the protein of the present invention is a disease where the level of the protein of the present invention is increased or decreased, as compared to the normal state.

Herein, the "disease where the level of the protein of the present invention is decreased, as compared to the normal state" includes, for example, schizophrenia, hypertension, burn, etc. (preferably schizophrenia, etc.).

The "disease where the level of the protein of the present invention is increased, as compared to the normal state" includes, for example, Parkinson's disease, motor function disorders (specifically, acute dystonic reaction, akathisia (akinesia), tardive dyskinesia (orofacial), distressful motor disturbances, disabling motor disturbances), depression, neurosis, generalized anxiety disorders, Alzheimer's disease, hepatitis (chronic hepatitis, fulminant hepatitis, subacute hepatitis, etc.), hepatic cirrhosis, hepatic fibrosis, diabetes mellitus, chronic heart failure, acromegaly, hyperthyroidism, psychosomatic disorders, etc. (preferably Parkinson's disease, etc.)

Furthermore, the disease which can be prevented/treated by promoting (enhancing) the activities (or functions; including "binding of PrRP and the PrRP receptor) of the protein of the present invention includes, for example, schizophrenia, hypertension, burn, etc. (preferably schizophrenia, etc.). On the other hand, the disease which can be prevented/treated by inhibiting (suppressing) the activities (or functions; including "binding of PrRP and the PrRP receptor) of the protein of the present invention includes, for example, Parkinson's disease, motor function disorders (specifically, acute dystonic reaction, akathisia (akinesia), tardive dyskinesia (orofacial), distressful motor disturbances, disabling motor disturbances), depression, neurosis, generalized anxiety disorders, Alzheimer's disease, hepatitis, hepatic cirrhosis, hepatic fibrosis, diabetes mellitus, chronic heart failure, acromegaly, hyperthyroidism, psychosomatic disorders, etc. (preferably Parkinson's disease, etc.).

The present invention relates to a method of screening a substance for the prevention/treatment of the disease associated with the protein, which comprises using the protein of the present invention.

The screening method of the present invention is performed, for example, by:
1) comparing the amounts of the protein of the present invention produced, in the case where a cell capable of producing the protein of the present invention (PrRP and/or the PrRP receptor; preferably the PrRP receptor) is incubated and in the case where a cell capable of producing the protein of the present invention is incubated in the presence of a test compound;
2) comparing the activities of the protein of the present invention, in the case where a cell capable of producing the protein of the present invention (PrRP and/or the PrRP receptor; preferably the PrRP receptor) is incubated and in the case where a cell capable of producing the protein of the present invention is incubated in the presence of a test compound (PrRP may be co-present);
3) comparing the activities of the protein of the present invention (PrRP and/or the PrRP receptor; preferably the PrRP receptor), in the cases where a test compound is present and absent; and so on.

The cell capable of producing the protein of the present invention is not particularly limited, so long as it is capable of producing the protein, preferably cells inducing the production of the protein of the present invention (preferably PrRP) in response to various stimuli including oxidative stress, a treatment with growth factor, etc.

The cell capable of producing the protein of the present invention may be the "transformant having DNA encoding the protein of the present invention" described above. Suitable examples of the cell capable of producing the protein of the present invention are cells isolated from mammal (preferably, human, rat, mouse, etc.), and the like. These cells may be immortalized.

The cell capable of producing the protein of the present invention may be incubated as in the transformant described above.

Examples of the test compound include peptides, proteins, non-peptide compounds, synthetic compounds, fermentation products, cell extracts, plant extracts, animal tissue extracts, etc.

The level of the protein of the present invention can be determined by publicly known methods, e.g., by using an antibody to the protein of the present invention, in accordance with methods like western blot analysis, ELISA, etc., or their modifications.

The antibody to the protein of the present invention may be any of monoclonal and polyclonal antibodies, as long as they are capable of recognizing the protein of the present invention. Furthermore, the antibody molecule per se may be used or F (ab')₂, Fab' or Fab fraction of the antibody molecule may also be used. The antibody may be labeled.

Examples of the labeling agent used in the assay method using the labeling substance are radioisotopes, enzymes, fluorescent substances and luminescent substances, etc. Examples of the radioisotope are [¹²⁵I], [¹³¹I], [³H], [¹⁴C], etc. Preferred examples of the enzyme are those that are stable and have a high specific activity, which include β-galactosidase, β-glucosidase, alkaline phosphatase, peroxidase and malate dehydrogenase. Examples of the fluorescent substance are fluorescamine, fluorescein isothiocyanate, etc. Examples of the luminescent substance are luminol, a luminol derivative, luciferin, lucigenin, etc. In addition, a biotin-avidin system may also be used for binding an antibody or antigen to a labeling agent.

In quantification of the protein of the present invention, the protein to be quantified may be any of those contained in cells or secreted outside cells, and may also be the sum of both.

When the protein of the present invention contained in cells is quantified, it is preferred to quantify after the cells are preferably treated in an appropriate fixing solution or with a treatment with a membrane-permeation promoter. Alternatively, after cells are suspended in an appropriate buffer, the cells are disrupted by ultrasonication, freeze-thaw, etc., followed by quantification of the protein in the lysate. If necessary, the protein may be quantified after the protein in the lysate is separated and purified

The activities of the protein of the present invention used in the screening method of the present invention include, for example, activation of dopamine neurons, etc. Specific examples are a dopamine-release promoting activity, a dopamine receptor agonizing activity, a monoamine oxidase-B inhibitory activity, and the like.

Examples of the polynucleotide include a polynucleotide comprising the base sequence represented by SEQ ID NO: 2, SEQ ID NO: 4, SEQ ID NO: 6 or SEQ ID NO: 8 later described (preferably, a polynucleotide comprising the base sequence represented by SEQ ID NO: 2, SEQ ID NO: 4, SEQ ID NO: 6 or SEQ ID NO: 8).

The activities described above can be measured in accordance with publicly known methods, e.g., a measurement method for a monoamine level using HPLC, or its modifications.

The substance for the prevention/treatment of the disease associated with the protein of the present invention, that is, the compound that modulates (promotes or inhibits) the production of the protein of the present invention or the compound that modulates (promotes (or enhances) or inhibits) the activity (or function) of the protein of the present invention can be screened by the screening method described above.

For example, a test compound that increases the level of the protein of the present invention by at least about 20%, preferably at least 30% and more preferably at least about 50% can be selected as the compound that promotes the production of the protein of the present invention; a test compound that decreases the level of the protein of the present invention by at least about 20%, preferably at least 30% and more preferably at least about 50% can be selected as the compound that inhibits the production of the protein of the present invention, respectively.

For example, a test compound that increases the activity of the protein of the present invention by at least about 20%, preferably at least 30% and more preferably at least about 50% can be selected as the compound that promotes activity of the protein of the present invention (preferably an agonist for the PrRP receptor); a test compound that decreases the activity of the protein of the present invention by at least about 20%, preferably at least 30% and more preferably at least about 50% can be selected as the compound that inhibits the activity of the protein of the present invention (preferably an antagonist to the PrRP receptor), respectively.

When an increase or decrease in the level of the protein in a subject is confirmed by quantifying the protein of the present invention, it can be diagnosed that the subject suffers from the disease associated with the protein of the present invention or it is highly likely that the subject might suffer from such a disease.

Also, when an increase or decrease in the activity of the protein in a subject is confirmed by assaying the activity of the present invention, it can be diagnosed that the subject suffers from the disease associated with the protein of the present invention, or it is highly likely that the subject might suffer from such a disease.

The substances for the prevention/treatment of the disease associated with the protein of the present invention, which are obtained by using the screening method of the present invention, include peptides, proteins, non-peptide compounds, synthetic compounds, fermentation products, cell extracts, plant extracts, animal tissue extracts, blood plasma, etc. These substances may form salts, and specific examples of these salts are the same as those given as the salts of the protein of the present invention described above.

The antibodies to the protein of the present invention can be manufactured according to publicly known methods for producing antibodies or antisera, using the protein of the present invention as antigens. Monoclonal antibodies or polyclonal antibodies to the protein of the present invention can be manufactured, for example, by the following procedures.

### [Preparation of Monoclonal Antibody]

### (a) Preparation of Monoclonal Antibody-Producing Cells

The protein of the present invention is administered to a mammal, either solely or together with carriers or diluents to the site that can produce the antibody by the administration. In order to potentiate the antibody productivity upon the administration, complete Freund's adjuvant or incomplete Freund's adjuvant may be administered. The administration is effected usually once every 2 to 6 weeks and approximately 2 to 10 times in total. The mammals to be used include simian, rabbit, dog, guinea pig, mouse, rat, ovine, goat, fowl and the like, with mouse and rat being preferred.

In the preparation of the monoclonal antibody-producing cells, an animal wherein the antibody titer is noted is selected from mammal immunized with antigens, e.g., mice, then spleen or lymph node is collected after two to five days from the final immunization and the antibody-producing cells contained therein are fused with myeloma cells to give monoclonal antibody-producing hybridomas. The antibody titer in antisera may be determined, for example, by reacting the labeled protein, which will be described later, with the antiserum followed by measuring the binding activity of the labeling agent bound to the antibody. The fusion may be carried out, for example, according to the method for Koehler and Milstein (Nature, 256, 495, 1975). Examples of the fusion accelerator are polyethylene glycol (PEG), Sendai virus, etc. and PEG is preferably used.

Examples of myeloma cells include NS-1, P3U1 and SP2/0, AP-1, etc. with P3U1 being preferred. The ratio of the number of the antibody-producing cells (spleen cells) to the number of myeloma cells to be used is preferably about 1:1 to about 20:1 and PEG (preferably PEG 1000 to PEG 6000) is added in a concentration of about 10% to about 80%. The cell fusion can be efficiently carried out by incubating both cells at about 20°C to about 40°C, preferably about 30°C to about 37°C for about 1 minute to about 10 minutes.

Various methods can be used for screening of a monoclonal antibody-producing hybridoma. Examples of such methods include a method which comprises adding the supernatant of hybridoma to a solid phase (e.g., microplate) adsorbed with the protein as antigen directly or together with a carrier, adding an anti-immunoglobulin antibody (where mouse cells are used for the cell fusion, anti-mouse immunoglobulin antibody is used) labeled with a radioactive substance, an enzyme, etc. or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance, an enzyme, etc. and detecting the monoclonal antibody bound to the solid phase; and the like.

The monoclonal antibody can be selected according to publicly known methods or their modifications. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1 % to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably 37°C, for about 5 days to about 3 weeks, preferably 1 to 2 weeks, normally in 5% CO₂. The antibody titer of the culture supernatant of a hybridoma can be determined as in the determination of antibody titer in antisera described above.

The monoclonal antibody thus obtained can be separated and purified by publicly known methods, for example, separation and purification of immunoglobulins, [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody in the antigen-binding solid phase or with an activated adsorbent such as Protein A, Protein G, etc. and dissociating the binding to obtain the antibody].

### [Preparation of Polyclonal Antibody]

The polyclonal antibody to the protein of the present invention can be manufactured by publicly known methods. For example, a complex of immunogen (protein antigen) and a carrier protein is formed and a mammal is immunized with the complex in a manner similar to the method described above for the manufacture of monoclonal antibody. The product containing the antibody to the protein of the present invention is collected from the immunized animal followed by separation and purification of the antibody.

In the complex of immunogen and carrier protein for immunizing mammals, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or keyhole limpet hemocyanin is coupled to hapten in a carrier-to-hapten weight ratio of approximately 0.1 to 20, preferably 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually carried out once every 2 to 6 weeks and 3 to 10 times in total.

The polyclonal antibody can be collected from the blood, ascites, etc., preferably from the blood of mammals immunized by the method described above.

The polyclonal antibody titer in antiserum can be determined by the same procedure as in the serum antibody titer described above. The polyclonal antibody can be separated and purified according to the same method for separation and purification of immunoglobulin as used for the monoclonal antibody described above.

The "substance (compound) for the prevention/treatment of the disease associated with the protein of the present invention," which is obtained by the screening method of the present invention, can be prepared into a pharmaceutical composition, if necessary, by mixing with a pharmacologically acceptable carrier, and used as a medicament for the prevention/treatment of the disease associated with the protein of the present invention.

Herein, examples of the pharmacologically acceptable carrier include various organic or inorganic carrier substances which are conventionally used as materials for pharmaceutical preparations. These carriers are incorporated as excipients, lubricants, binders and disintegrants in solid preparations; and, as solvents, solubilizers, suspending agents, isotonizing agents, buffers, soothing agents, etc. in liquid preparations. Depending on necessity, additives such as preservatives, antioxidants, coloring agents, sweetening agents, etc. may also be used.

Preferred examples of the excipients include lactose, sucrose, D-mannitol, D-sorbitol, starch, gelatinized starch, dextrin, crystalline cellulose, low-substituted hydroxypropylcellulose, carboxymethylcellulose sodium, gum arabic, dextrin, pullulan, light silicic anhydride, synthetic aluminum silicate, magnesium aluminometasilicate, etc.

Preferred examples of the lubricants include magnesium stearate, calcium stearate, talc, colloidal silica, etc.

Preferred examples of the binders include gelatinized starch, saccharose, gelatin, gum arabic, methylcellulose, carboxymethylcellulose, carboxymethylcellulose sodium, crystalline cellulose, sucrose, D-mannitol, trehalose, dextrin, pullulan, hydroxypropylcellulose, hydroxypropylmethylcellulose, polyvinylpyrrolidone, etc.

Preferred examples of the disintegrants include lactose, sucrose, starch, carboxymethylcellulose, carboxymethylcellulose calcium, croscarmellose sodium, carboxymethylstarch sodium, light silicic anhydride, low-substituted hydroxypropylcellulose, etc.

Preferred examples of the solvents include water for injection, physiological saline, Ringer's solution, alcohol, propylene glycol, polyethylene glycol, sesame oil, corn oil, olive oil, cotton seed oil, etc.

Preferred examples of the solubilizers include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate, etc.

Preferred examples of the suspending agents include surfactants such as stearyltriethanolamine, sodium lauryl sulfate, laurylaminopropionic acid, lecithin, benzalkonium chloride, benzethonium chloride, glycerol monostearate, etc.; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose, etc.; polysorbates, polyoxyethylene-hardened castor oil, etc.

Preferred examples of the isotonizing agents include sodium chloride, glycerol, D-mannitol, D-sorbitol, glucose, etc.

Preferred examples of the buffers include buffer solutions of phosphate, acetate, carbonate, citrate, etc.

Preferred examples of the soothing agents include benzyl alcohol, etc.

Preferred examples of the preservatives include para-hydroxybenzoate esters, chlorobutanol, benzyl alcohol, phenethyl alcohol, dehydroacetic acid, sorbic acid, etc.

Preferred examples of the antioxidants include sulfite salts, ascorbate salts, etc.

Preferred examples of the coloring agents include water-soluble colored tar dyes (e.g. food colors such as Food Color Red No. 2 and No. 3, Food Color Yellow No. 4 and No. 5, Food Color Blue No. 1 and No. 2, etc.), water-insoluble lake colors (e.g. aluminum salts of the above water-soluble edible tar colors, etc.), natural colors (e.g. β-carotene, chlorophyll, colcothar, etc.).

Preferred examples of the sweetening agents include saccharin sodium, dipotassium glycyrrhizinate, aspartame, stevia, etc.

Dosage forms of the pharmaceutical composition described above include oral preparations such as tablets, capsules (including soft capsules and microcapsules), granules, powders, syrups, emulsions, suspensions; and parenteral preparations such as injections (e.g., subcutaneous injection, intravenous injection, intramuscular injection, intraperitoneal injection, etc.), external application forms (e.g., nasal preparations, transdermal preparations, ointments, etc.), suppositories (e.g., rectal suppositories, vaginal suppositories, etc.), pellets, drip infusions, sustained-release preparations (e.g., sustained-release microcapsules, etc.). These preparations can be safely administrated orally or parenterally, respectively.

The pharmaceutical composition can be produced by conventional methods in the field of pharmaceutical manufacturing techniques, for example, methods described in the Japanese Pharmacopoeia. Specific methods for producing pharmaceutical preparations are described below in detail. The content of the compound in the pharmaceutical composition, which is obtained by the screening method of the present invention, may vary depending on the dosage form, dose of the compound, etc. and is approximately 0.1 to 100 wt%.

An oral preparation, for instance, is produced by adding to the active ingredient an excipient (e.g., lactose, sucrose, starch, D-mannitol, etc.), a disintegrant (e.g., carboxymethylcellulose calcium, etc.), a binder (e.g., gelatinized starch, gum arabic, carboxymethylcellulose, hydroxypropylcellulose, polyvinylpyrrolidone, etc.) or a lubricant (e.g., talc, magnesium stearate, polyethyleneglycol 6000, etc.) or the like, compression molding the obtained mixture, then, if necessary coating by a per se known method using a coating base for the purpose of taste masking, enteric coating or sustained release by techniques per se publicly known.

Examples of the coating base include a sugar coating base, a water-soluble film coating base, an enteric film coating base, a sustained-release film coating base, etc.

As the sugar coating base sucrose is employed. Further, one or two or more species selected from talc, precipitated calcium carbonate, gelatin, gum arabic, pullulan, carnauba wax and the like may be used in combination.

Examples of the water-soluble film coating base include cellulose polymers such as hydroxypropylcellulose, hydroxypropylmethylcellulose, hydroxyethylcellulose, methylhydroxyethylcellulose, etc.; synthetic polymers such as polyvinylacetal diethylaminoacetate, aminoalkyl methacrylate copolymer E [Eudragit E (trademark), Rohm Pharma], polyvinylpyrrolidone, etc.; polysaccharides such as pullulan, etc.

Examples of the enteric film coating base include cellulose polymers such as hydroxypropylmethylcellulose phthalate, hydroxypropylmethylcellulose acetate succinate, carboxymethylethylcellulose, cellulose acetate phthalate, etc.; acrylic acid polymers such as methacrylic acid copolymer L [Eudragit L (trademark), Rohm Pharma], methacrylic acid copolymer LD [Eudragit L-30D55 (trademark), Rohm Pharma], methacrylic acid copolymer S [Eudragit S (trademark), Rohm Pharma], etc.; natural products such as shellac and the like.

Examples of the sustained-release film coating base include cellulose polymers such as ethylcellulose, etc.; acrylic acid polymers such as aminoalkyl methacrylate copolymer RS [Eudragit RS (trademark), Rohm Pharma] and an ethyl acrylate-methyl methacrylate copolymer suspension [Eudragit NE (trademark), Rohm Pharma], etc.

Two or more of the above coating bases may be used in admixture in an appropriate ratio. On the occasion of coating, a shading agent such as titanium oxide, red ferric oxide may be used.

Injections are produced by dissolving, suspending or emulsifying the active ingredient in an aqueous solvent (e.g., distilled water, physiological saline, Ringer's solution, etc.) or an oleaginous solvent (e.g., vegetable oils such as olive oil, sesame oil, cotton seed oil, corn oil, etc.; propylene glycol, etc.), together with a dispersant (e.g., polysorbate 80, polyoxyethylene-hardened castor oil 60, etc., polyethylene glycol, carboxymethylcellulose, sodium alginate, etc.), a preservative (e.g., methylparaben, propylparaben, benzyl alcohol, chlorobutanol, phenol, etc.), an isotonizing agent (e.g., sodium chloride, glycerol, D-mannitol, D-sorbitol, glucose, etc.) and the like. If desirable, additives such as a solubilizer (e.g., sodium salicylate, sodium acetate, etc.), a stabilizer (e.g., human serum albumin, etc.), a soothing agents (e.g., benzyl alcohol, etc.) or the like, may be used. The thus-prepared liquid for injection is normally filled in an appropriate ampoule.

Since the thus obtained pharmaceutical preparation is safe and low toxic, and can be administered orally or parenterally to mammals (e.g., human, mice, rats, rabbits, sheep, swine, bovine, horses, fowl, cats, dogs, monkeys, chimpanzee, etc.).

The dose of the substance for the prevention/treatment of the disease associated with the protein of the present invention varies depending on target disease, subject to be administered, route for administration, etc.; the dose is normally about 0.1 to about 100 mg, preferably about 1.0 to about 50 mg, and more preferably about 1.0 to about 20 mg per day for an adult patient with Parkinson's disease (weighing 60 kg) as the compound (antagonist, etc.) obtained by the screening method of the present invention, which is the active ingredient.

The antibody to the protein of the present invention can also be used as a medicament for the prevention/treatment of the disease associated with the protein of the present invention.

The medicament for the prevention/treatment can be the antibody itself to the protein of present invention, but preferably is in the form of a pharmaceutical composition obtained by mixing the antibody with pharmacologically acceptable carriers. Herein, the pharmacologically acceptable carriers include those given for "the substance for the prevention/treatment of the disease associated with the protein of the present invention" described above.

The pharmaceutical composition can be manufactured in a manner similar to the method for preparing "the substance for the prevention/treatment of the disease associated with the protein of the present invention" described above.

Since the thus obtained pharmaceutical preparation is safe and low toxic, and can be administered orally or parenterally to mammals (e.g., human, mice, rats, rabbits, sheep, swine, bovine, horses, fowl, cats, dogs, monkeys, chimpanzee, etc.).

The dose of the substance for the prevention/treatment of the disease associated with the protein of the present invention varies depending on target disease, subject to be administered, route for administration, etc.; the dose is normally about 0.1 to about 100 mg, preferably about 1.0 to about 50 mg, and more preferably about 1.0 to about 20 mg per day for an adult patient with Parkinson's disease (weighing 60 kg) as the antibody to the protein of the present invention (the antibody to PrRP or the PrRP receptor (preferably, neutralizing antibody), etc.), which is the active ingredient.

The present invention further relates to a method of screening the substance for the prevention/treatment of the disease associated with the protein of the present invention, which comprises using a polynucleotide encoding the protein of the present invention (hereinafter sometimes briefly referred to as the polynucleotide of the present invention).

The polynucleotide of the present invention may be any polynucleotide so long as it contains the base sequence (DNA or RNA, preferably DNA) encoding the protein of the present invention. The polynucleotide may be DNA encoding the protein of the present invention, RNA such as mRNA, etc. The polynucleotide may be either double-stranded or single-stranded. When the polynucleotide is double-stranded, it may be double-stranded DNA, double-stranded RNA or a DNA:RNA hybrid. When the polynucleotide is single-stranded, it may be a sense strand (i.e., coding strand) or an antisense strand (i.e., non-coding strand). The DNA encoding the protein of the present invention includes the DNA described above.

The screening method of the present invention is performed by, for example, comparing the levels of the polynucleotide encoding the protein of the present invention, in the case where a cell capable of producing the protein of the present invention (PrRP and/or the PrRP receptor; preferably the PrRP receptor) is incubated and in the case where a cell capable of producing the protein of the present invention (PrRP and/or the PrRP receptor; preferably the PrRP receptor) is incubated in the presence of a test compound; or the like.

Herein, the cell capable of producing the protein of the present invention, methods for incubation of the cell and the test compound are the same as those given for the aforesaid screening method using the protein of the present invention.

The polynucleotide encoding the protein of the present invention can be quantified according to publicly known methods, for example, the method described in Molecular Cloning, 2nd (J. Sambrook et al., Cold Spring Harbor Lab. Press, 1989) or its modifications. For instance, when the polynucleotide is mRNA, the mRNA can be quantified by publicly known methods, for example, northern hybridization using as a probe a nucleic acid of SEQ ID NO: 2, SEQ ID NO: 4, SEQ ID NO: 6 or SEQ ID NO: 8, or containing a part thereof; PCR using as a probe a nucleic acid of SEQ ID NO: 2, SEQ ID NO: 4, SEQ ID NO: 6 or SEQ ID NO: 8, or containing a part thereof, or its modifications; or the like.

For example, a test compound that increases the level of the polynucleotide (preferably mRNA) encoding the protein of the present invention by at least about 20%, preferably at least 30% and more preferably at least about 50% can be selected as the compound that promotes the expression of the polynucleotide (preferably mRNA) encoding the protein of the present invention; a test compound that decreases the level of the polynucleotide encoding the protein of the present invention by at least about 20%, preferably at least 30% and more preferably at least about 50% can be selected as the compound that inhibits the expression of the polynucleotide encoding the protein of the present invention, respectively.

The substances for the prevention/treatment of the disease associated with the protein of the present invention, which are obtained by the screening method using the polynucleotide of the present invention, may be any of peptides, proteins, non-peptide compounds, synthetic compounds, fermentation products, cell extracts, plant extracts, animal tissue extracts, blood plasma, etc. These substances may form salts, and specific examples of these salts are the same as those given as the salts of the protein of the present invention described above.

The substance for the prevention/treatment of the disease associated with the protein of the present invention, which is obtained by the screening method, can be prepared into a pharmaceutical composition, if necessary, by mixing the substance with pharmacologically acceptable carriers, and used as a medicament for the prevention/treatment of the disease associated with the protein of the present invention.

Herein, the pharmacologically acceptable carriers include those given for the substance for the prevention/treatment of the disease associated with the protein of the present invention, which is obtained by the screening method using the protein of the present invention.

The pharmaceutical composition can be manufactured in a manner similar to the method for preparing the substance for the prevention/treatment of the disease associated with the protein of the present invention, which is obtained by the screening method using the protein of the present invention.

Since the thus obtained pharmaceutical preparation is safe and low toxic, and can be administered orally or parenterally to mammals (e.g., human, mice, rats, rabbits, sheep, swine, bovine, horses, fowl, cats, dogs, monkeys, chimpanzee, etc.).

The dose of the substance for the prevention/treatment of the disease associated with the protein of the present invention varies depending on target disease, subject to be administered, route for administration, etc.; the dose is normally about 0.1 to about 100 mg, preferably about 1.0 to about 50 mg, and more preferably about 1.0 to about 20 mg per day for an adult patient with Parkinson's disease (weighing 60 kg) as the compound obtained by the screening method of the present invention, which is the active ingredient.

The substance for the prevention/treatment of the disease associated with the protein of the preset invention can also be screened by detecting the promoter activity of DNA encoding the protein.

In the cell or non-human mammal in which the DNA encoding the protein of the present invention is replaced by a reporter gene, the reporter gene is present under control of a promoter for the DNA encoding the protein of the present invention. Thus, the activity of the promoter can be detected by confirming that a substance encoded by the reporter gene is expressed after a test compound is treated or administered.

The activity of the promoter can be detected also in the cell and non-human mammal bearing a vector constructed by ligating the transcriptional regulatory domain of the DNA encoding the protein of the present invention and the reporter gene.

Herein, examples of the reporter gene are β-galactosidase gene (lacZ), soluble alkaline phosphatase gene, luciferase gene, and the like.

When a part of the DNA region encoding the protein of the present invention is substituted with, e.g., β-galactosidase gene (lacZ) derived from *Escherichia coli,* β-galactosidase is expressed in a tissue where the protein of the present invention should originally be expressed, instead of the protein of the present invention. Thus, the expression state of the protein of the present invention can be readily confirmed by staining with a reagent, e.g., 5-bromo-4-chloro-3-indolyl-β-galactopyranoside (X-gal) which is a substrate for β-galactosidase. Specifically, a cell or tissue section is fixed with glutaraldehyde, etc. After washing with phosphate buffered saline (PBS), the reaction is carried out with a staining solution containing X-gal at room temperature or about 37°C for approximately 30 minutes to an hour. The β-galactosidase reaction is then terminated by washing the tissue preparation with 1 mM EDTA/PBS solution, and the color formed is observed, whereby the expression state of the protein of the present invention in the cell or tissue can be confirmed. Alternatively, mRNA encoding lacZ may be detected in a conventional manner.

The compound that promotes or inhibits the activity of a promoter for the DNA encoding the protein of the present invention is useful as the medicament for the prevention/treatment of the disease associated with the protein of the present invention because the compound modulates the expression of the protein and the activity of the protein.

The protein of the present invention is increasingly expressed in, e.g., Parkinson's disease, etc. and is thus useful as a marker for early diagnosis in Parkinson's disease, judgment of the severity in conditions and prognosis of progress of the disease. Accordingly, the antibody to the protein of the present invention, the antisense nucleotide of the DNA encoding the protein of the present invention are useful as the medicament for the prevention/treatment of the disease associated with the protein of the present invention, as in the compound obtained by the screening method of the present invention.

Herein, the antisense nucleotide can be any nucleotide, as far as it has a base sequence complementary or substantially complementary to the base sequence of DNA encoding the protein of the present invention and possesses an action of suppressing the expression of the DNA, but an antisense DNA is preferred.

The base sequence substantially complementary to the base sequence of the DNA encoding the protein of the present invention includes, for example, a base sequence having at least about 70% homology, preferably at least about 80% homology, more preferably at least about 90% homology and most preferably at least about 95% homology, to the entire base sequence or to its partial base sequence (i.e., complementary strand to the DNA encoding the protein of the present invention), and the like. Especially in the entire base sequence of the complementary strand to the DNA encoding the protein of the present invention, preferred are an antisense nucleotide having at least about 70% homology, preferably at least about 80% homology, more preferably at least about 90% homology and most preferably at least about 95% homology, to the complementary strand of the base sequence which encodes the N-terminal region of the protein (e.g., the base sequence around the initiation codon, etc.).

Specific examples include an antisense nucleotide comprising the entire or part of a base sequence complementary or substantially complementary to the base sequence of DNA comprising the base sequence represented by SEQ ID NO: 2, SEQ ID NO: 4, SEQ ID NO: 6 or SEQ ID NO: 8, preferably an antisense nucleotide comprising the entire or part of a base sequence complementary to the base sequence of DNA comprising the base sequence represented by SEQ ID NO: 2, SEQ ID NO: 4, SEQ ID NO: 6 or SEQ ID NO: 8, more preferably an antisense nucleotide comprising the entire or part of a base sequence complementary to the base sequence of DNA comprising the base sequence represented by SEQ ID NO: 2, SEQ ID NO: 4, SEQ ID NO: 6 or SEQ ID NO: 8; and the like.

The antisense nucleotide is generally constituted by bases of about 10 to about 40, preferably about 15 to about 30.

To prevent digestion with a hydrolase such as nuclease, etc., the phosphoric acid residue (phosphate) of each nucleotide that constructs the antisense nucleotide may be substituted with chemically modified phosphoric acid residues, e.g., phosphorothioate, methyl phosphonate, phosphorodithionate, etc. Also, the sugar (deoxyribose) in each nucleotide may be replaced by a chemically modified structure such as 2'-O-methylation, etc. The base part (pyrimidine, purine) may also be chemically modified and may be any one which hybridizes to a DNA comprising the base sequence represented by SEQ ID NO: 2. These antisense nucleotides may be synthesized using a publicly known DNA synthesizer, etc.

The antisense nucleotide of the DNA encoding the protein of the present invention is low toxic and can suppress the function of the protein of the present invention or the function of the DNA encoding the protein (e.g., a thyroxine 5'-deiodinase activity) in vivo, and can thus be used as a medicament for the prevention/treatment of the disease associated with the protein of the present invention. The antisense nucleotide can be prepared into pharmaceutical preparations as in the substance for the prevention/treatment of the disease associated with the protein of the present invention, which is obtained by the screening method using the protein of the present invention. These pharmaceutical preparations can be administered orally or parenterally to mammals (e.g., human, mice, rats, rabbits, sheep, swine, bovine, horses, fowl, cats, dogs, monkeys, chimpanzees, etc.). Also, the antisense nucleotide can be administered after it is inserted into an appropriate vector, e.g., retrovirus vector, adenovirus vector, adenovirus-associated virus vector, etc.

The antisense nucleotide may also be administered by gene gun or through a catheter such as a catheter with a hydrogel. Alternatively, the antisense nucleotide may be prepared into an aerosol, which is topically administered into the trachea as an inhaler.

The dose of the antisense nucleotide varies depending on target disease, subject to be administered, route for administration, etc.; the dose is normally about 0.1 to about 100 mg, preferably about 1.0 to about 50 mg, and more preferably about 1.0 to about 20 mg per day for an adult patient with Parkinson's disease (weighing 60 kg).

In addition, the antisense nucleotide of DNA encoding the protein of the present invention may also be used as an oligonucleotide probe for diagnosis to examine the presence of the DNA in tissues or cells and the state of its expression.

In addition, the present invention provides the following features.
(1) A double-stranded RNA comprising a part of the RNA encoding the protein of the present invention.
(2) A medicament comprising the double-stranded RNA described above.
(3) A ribozyme comprising a part of the RNA encoding the protein of the present invention.
(4) A medicament comprising the ribozyme described above.

As the antisense nucleotide described above can, double-stranded RNA (siRNA or RNAi; the RNA interference method), ribozyme, etc. can suppress the expression of the polynucleotide (e.g., DNA) encoding the protein of the present invention and can suppress the in vivo function of the protein or DNA of the present invention, and the function of the peptide of the present invention depending on the same. Thus, they can be used as agents for the prevention/treatment of, for example, Parkinson's disease, motor function disorders (specifically, acute dystonic reaction, akathisia (akinesia), tardive dyskinesia (orofacial), distressful motor disturbances, disabling motor disturbances), depression, neurosis, generalized anxiety disorders, Alzheimer's disease, hepatitis (chronic hepatitis, fulminant hepatitis, subacute hepatitis, etc.), hepatic cirrhosis, hepatic fibrosis, diabetes mellitus, chronic heart failure, acromegaly, hyperthyroidism, psychosomatic disorders, etc.

Herein, the double-stranded RNA can be designed based on a sequence of the polynucleotide of the present invention and manufactured by modifications of publicly known methods, for example, the method described in Nature, 411, 494, 2001.

The ribozyme can be designed based on a sequence of the polynucleotide of the present invention and manufactured by modifications of publicly known methods, e.g., the method described in TRENDS in Molecular Medicine, 7, 221, 2001. For example, the ribozyme can be manufactured by ligating a publicly known ribozyme to a part of the RNA encoding the protein of the present invention. A part of the RNA encoding the protein of the present invention includes a portion in the vicinity of a cleavage site on the RNA encoding the protein of the present invention, which may be cleaved by a publicly known ribozyme (RNA fragment).

Where the double-stranded RNA or ribozyme described above can be used as the medicament for the prevention/treatment of the disease associated with the protein of the present invention, the ribozyme can be prepared into pharmaceutical preparations, which are provided for administration, as in the antisense polynucleotide.

By using the polynucleotide of the present invention, e.g., as a probe, an abnormality (gene abnormality) of the DNA or mRNA encoding the protein of the present invention in mammal (e.g., human, rat, mouse, guinea pig, rabbit, fowl, sheep, swine, bovine, horse, cat, dog, monkey, chimpanzee, etc.) can be detected. Therefore, the DNA is useful as a gene diagnostic agent for damages to the DNA or mRNA, its mutation or decreased expression, or increased expression or overexpression of the DNA or mRNA, etc.

The gene diagnosis described above using the DNA encoding the protein of the present invention can be performed by, for example, publicly known northern hybridization or PCR-SSCP (Genomics, 5, 874-879 (1989); Proceedings of the National Academy of Sciences of the United States of America, 86, 2766-2770 (1989)), etc.

For example, when overexpression or reduced expression is detected by the northern hybridization or DNA mutation is detected by the PCR-SSCP, it can be diagnosed that it is highly likely to suffer from the disease associated with the protein of the present invention, such as schizophrenia, Parkinson's disease, etc.

The present invention relates to the medicament for the prevention/treatment of the disease associated with the protein of the present invention, which comprises the polynucleotide comprising the base sequence represented by SEQ ID NO: 2, SEQ ID NO: 4, SEQ ID NO: 6 or SEQ ID NO: 8. Herein, the polynucleotide comprising the base sequence represented by SEQ ID NO: 2, SEQ ID NO: 4, SEQ ID NO: 6 or SEQ ID NO: 8 is a decoy nucleotide against the DNA, to which the protein of the present invention binds.

The polynucleotide can be manufactured in accordance with publicly known methods.

The polynucleotide is low toxic and can suppress the function of the protein of the present invention or the function of the DNA encoding the protein (e.g., a thyroxine 5'-deiodinase activity) in vivo, and can thus be used as a medicament for the prevention/treatment of the disease associated with the protein of the present invention. The polynucleotide comprising the base sequence represented by SEQ ID NO: 5 or SEQ ID NO: 6 can be prepared into pharmaceutical preparations as in the substance for the prevention/treatment of the disease associated with the protein of the present invention, which is obtained by the screening method using the protein of the present invention. These pharmaceutical preparations can be administered orally or parenterally to mammals (e.g., human, mice, rats, rabbits, sheep, swine, bovine, horses, fowl, cats, dogs, monkeys, chimpanzees, etc.).

Also, the polynucleotide can be administered after the polynucleotide is inserted into an appropriate vector such as retrovirus vector, adenovirus vector, adenovirus-associated virus vector, etc.

The polynucleotide can also be administered by gene gun or through a catheter such as a catheter with a hydrogel. Alternatively, the polynucleotide may be prepared into an aerosol, which is topically administered into the trachea as an inhaler.

The dose of the medicament for the prevention/treatment of the disease associated with the protein of the present invention varies depending on target disease, subject to be administered, route for administration, etc.; the dose is normally about 0.1 to about 100 mg, preferably about 1.0 to about 50 mg, and more preferably about 1.0 to about 20 mg per day for an adult patient with Parkinson's disease (weighing 60 kg), as the polynucleotide comprising the base sequence represented by SEQ ID NO: 5 or SEQ ID NO: 6, which is the active ingredient.

The present invention also relates to a medicament for the prevention/treatment of Parkinson's disease, which comprises the PrRP suppressor.

The PrRP suppressor is not particularly limited, so long as it is a substance capable of suppressing the production or expression of PrRP; or capable of suppressing the activity of PrRP, and can be any one of a peptide, a protein, a non-peptide compound, a synthetic compound, a fermentation product, a cell extract, a plant extract, an animal tissue extract, plasma, etc. These substances may form salts, and specific examples of these salts are the same as those given as the salts of the protein of the present invention described above.

The medicament for the prevention/treatment of Parkinson's disease can be prepared into pharmaceutical preparations using the PrRP suppressor, in the same manner as in the substance for the prevention/treatment of the disease associated with the protein of the present invention Since the medicament of the present invention for the prevention/treatment of Parkinson's disease is safe and low toxic, and can be administered orally or parenterally to mammals (e.g., human, mice, rats, rabbits, sheep, swine, bovine, horses, fowl, cats, dogs, monkeys, chimpanzee, etc.).

The dose of the medicament of the present invention for the prevention/treatment of Parkinson's disease varies depending on target disease, subject to be administered, route for administration, etc.; the dose is normally about 0.1 to about 100 mg, preferably about 1.0 to about 50 mg, and more preferably about 1.0 to about 20 mg per day for an adult patient (weighing 60 kg), as the PrRP suppressor, which is the active ingredient.

By using the PrRP-encoding DNA, e.g., as a probe, an abnormality (gene abnormality) of the PrRP-encoding DNA or mRNA in human or other warm-blooded animal (e.g., rat, mouse, guinea pig, rabbit, fowl, sheep, swine, bovine, horse, cat, dog, monkey, etc.) can be detected. Therefore, the DNA is useful as a gene diagnostic agent for damages to the DNA or mRNA, its mutation or decreased expression, or increased expression or overexpression of the DNA or mRNA; etc.

The gene diagnosis described above using the DNA of the present invention can be performed by, for example, the publicly known northern hybridization, PCR-SSCP (Genomics, 5, 874-879, 1989); Proceedings of the National Academy of Sciences of the United States of America, 86, 2766-2770, 1989), the DNA microarray, etc.

In the specification, when the bases and amino acids are expressed by codes, the codes are denoted in accordance with the IUPAC-IUB Commission on Biochemical Nomenclature or by the conventional codes in the art, examples of which are shown below. For amino acids that may have the optical isomer, L form is presented, unless otherwise indicated.
- DNA: : deoxyribonucleic acid
- cDNA: : complementary deoxyribonucleic acid
- A: : adenine
- T: : thymine
- G: : guanine
- C: : cytosine
- RNA: : ribonucleic acid
- mRNA: : messenger ribonucleic acid
- dATP: : deoxyadenosine triphosphate
- dTTP: : deoxythymidine triphosphate
- dGTP: : deoxyguanosine triphosphate
- dCTP: : deoxycytidine triphosphate
- ATP: : adenosine triphosphate
- EDTA: : ethylenediaminetetraacetic acid
- SDS: : sodium dodecyl sulfate
- Gly: : glycine
- Ala: : alanine
- Val: : valine
- Leu: :leucine
- Ile: : isoleucine
- Ser: : serine
- Thr: : threonine
- Cys: : cysteine
- Met:: methionine
- Glu: : glutamic acid
- Asp: : aspartic acid
- Lys: :lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu: : pyroglutamic acid

The sequence identification numbers (SEQ ID NO:) in the sequence listing of the specification indicates the following sequence, respectively.
[SEQ ID NO: 1]
   This shows the amino acid sequence of human PrRP.
[SEQ ID NO: 2]
   This shows the base sequence of cDNA encoding human PrRP.
[SEQ ID NO: 3]
   This shows the amino acid sequence of mouse PrRP.
[SEQ ID NO: 4]
   This shows the base sequence of DNA encoding mouse PrRP.
[SEQ ID NO: 5]
   This shows the amino acid sequence of rat PrRP.
[SEQ ID NO: 6]
   This shows the base sequence of cDNA encoding rat PrRP.
[SEQ ID NO: 7]
   This shows the amino acid sequence of bovine PrRP.
[SEQ ID NO: 8]
   This shows the base sequence of cDNA encoding bovine PrRP.
[SEQ ID NO: 9]
   This shows the amino acid sequence of human PrRP receptor.
[SEQ ID NO: 10]
   This shows the base sequence of cDNA encoding human PrRP receptor.

Hereinafter, the present invention will be described specifically by referring to EXPERIMENTAL EXAMPLES but is not deemed to be limited thereto.

### EXAMPLE 1

### Effect on PrRP Knockout Mice by Administration of MPTP (methyl-phenyl-tetrahydopyridine)

MPTP (methyl-phenyl-tetrahydopyridine) known to produce animal models of Parkinson's disease was intraperitoneally administered to PrRP knockout mice and wild-type mice every 2 hours 4 times at the dose of 20 µg/kg. Five days after the administration, 11 out of 21 wild-type mice survived with a survival rate of 52%, whereas 10 out of 10 PrRP knockout mice survived, meaning that there was no dead animal and the survival rate was 100%. These results suggest that PrRP knockout mice are extremely tolerant to MPTP.

### EXAMPLE 2

### Effect on Nigral Dopamine Cell of PrRP Knockout Mice by Administration of MPTP (methyl-phenyl-tetrahydopyridine)

It is known that MPTP (methyl-phenyl-tetrahydopyridine) causes degenerative loss of nigral dopamine cells to induce Parkinson's disease. Thus, the brains were excised from PrRP knockout mice and wild-type mice, to which MPTP or saline for control was given as in EXAMPLE 1, and then immersed and fixed in a 0.07M phosphate buffer (pH 7.4) containing 5% acrolein (Tokyo Chemical Industry). The brains were then immersed in 30% sucrose-containing phosphate buffered saline (PBS) for more than 2 days. The free-floating technique was used for immunohistochemistry. A frozen frontal section in 40 µm thickness of the brain was prepared using a cryomicrotome. Next, the section was soaked in and washed with PBS for water immersion, then treated with 0.5% sodium metaperiodate-containing PBS (20 minutes) to inactivate the endogenous peroxidase activity, and the aldehyde group was reduced by treatment with 1% sodium borohydride-containing PBS (20 minutes). In addition, non-specific reactions were blocked by treatment with 1% normal horse serum and 0.4% Triton X-100-containing BPS (TNBS) (an hour). Localization of dopamine cells was visualized by the avidin biotinylated-horseradish-peroxidase (HRP) complex (ABC) method (Vector Laboratory) using the presence of tyrosine hydroxylase as an indicator. In other words, the section was reacted with TNBS containing TH2 (Sigma) (40 µg/ml), which is a specific monoclonal antibody to tyrosine hydroxylase, at room temperature for 12 hours or longer. After thoroughly washing with PBS, the section was reacted with biotin-conjugated donkey anti-mouse IgG (Chemicon International)-containing TNBS (1:300) at room temperature for 2 hours. After thoroughly washing with PBS, the section was reacted with ABC at room temperature for 30 minutes, followed by color-forming reaction using as substrate 50 mM Tris-HCl (pH 7.6) containing 0.02% 3,3-diaminobenzidine-tetrachloride (Dojin Chemical Laboratory) and 0.006% H₂O₂. After the color formation was terminated by washing with PBS, the section was mounted on a slide glass, dehydrated, penetrated with xylene and sealed in Entellan (Merck). The number of tyrosine hydroxylase positive cells, which are dopamine cells in the substantia nigra, was counted under an optical microscope.

In the wild-type mice, a significant decrease by about 60% was observed in the MPTP group, as compared to the saline group, whereas in the PrRP knockout mice, the decrease in the MPTP group was about 10% as compared to the saline group, indicating that there was no significant change (FIG 1).

### EXAMPLE 3

### Effect on Motor Activity in PrRP Knockout Mice by Administration of MPTP (methyl-phenyl-tetrahydopyridine)

In order to examine the relation of Parkinson's disease to PrRP, decrement of motor activities known as symptoms of Parkinson's disease induced by administration of MPTP was compared between PrRP knockout mice and wild-type mice. The motor activity of each mouse was assessed with a rotarod. In the wild-type mice, the motor activity was markedly decreased in the group administered with MPTP, as compared to the group administered with saline, and it was confirmed that symptoms like Parkinson's disease were induced. Turning to the PrRP knockout mice, there was no difference between the group administered with MPTP and the group administered with saline for control, and the motor activity was normal (FIG. 2). These phenomena reveal that onset of Parkinson's disease induced by MPTP was suppressed in the PrRP knockout mice.

### EXAMPLE 4

### Measurement of Catecholamine Level in Brain of PrRP Knockout Mice by Administration of MPTP (methyl-phenyl-tetrahydopyridine)

MPTP or saline for control was administered to PrRP knockout mice and wild-type mice in a manner similar to EXAMPLE 1. Seven days after the final administration, the striatal sites were immediately excised from the brains, frozen in liquid nitrogen and then stored at -80°C. Catecholamines such as Dopamine (DA), DOPAK, HVA, etc. were measured as follows. First, the excised corpus striata were homogenized in 0.2M perchloric acid containing 100 ng/mL isoproterenol (Sigma), centrifuged at 15,000 g for 20 minutes at 0°C, and filtered through a 0.22 µm filter. The final solution, 10 µL, was separated under the conditions at flow rate of 0.5 mL/min in 83% 0.1 M acetic acid - citrate buffer (pH 3.5)/17 % methanol solution containing 190 mg/L SDS and 5 mg/L EDTA·2Na through EICOMPAK SC-50DS column (3.0 mm x 150 mm, EICOM), and determined by electrochemical detection. In the wild-type mice, dopamine (DA), homogentisic acid (DOPAC) and homovanillic acid (HVA), which are catecholamines, were all decreased markedly by administration of MPTP, whereas the degree of the decrease was smaller in the PrRP knockout animal (FIG. 3).

### EXAMPLE 5

### Effects of Administration of MPTP (methyl-phenyl-tetrahydopyridine) on Nigral Dopamine Cell and Striatal Dopamine Nerve Fiber after Administration of Anti-PrRP Neutralizing Antibody (P2L-1Ca; see EP-A-1081222)

Male C57BL/6J mouse (CLEA Japan) of 14 weeks old was fixed on a brain stereotaxic apparatus under anesthesia with pentobarbital (Dainippon Pharmaceutical) and the skull surface was exposed. After the skull was dried with a drier, the coordinates of bregma were located to determine the coordinates of the left lateral ventricle (AP: -0.2 mm, lateral: +1.0 mm, depth: +2.5 mm from bregma). After a burr hole was made with a dental drill on the skull across the bregma from the location of the left lateral ventricle, anchor screws (EICOM) were implanted to fix a dental cement (GC Corp., Unifast II). A hole was made with a dental drill (Urawa Corp., Model: UC100) for guide cannulation to vertically insert into the skull on the left lateral ventricle, and a guide cannula (EICOM) was eased down toward the ventricle. After leakage of cerebrospinal fluids was confirmed, the guide cannula was fixed on the coordinates determined. The dental cement was used for fixation and applied until the anchor screws were embedded. After it was confirmed that the cement was dried, the mouse was released from the stereotaxic apparatus. A dummy cannula was inserted into the guide cannula (EICOM) and fixed with a cap nut (EICOM). During the one-week recovery period, the animal was observed for behaviors and received handling to habituate.

The antibodies used were normal mouse IgG (Sigma) for control antibody or anti-PrRP neutralizing antibody P2L-1Ca. The antibody solution was diluted in Centriprep (Millipore). Absorbance (280 nm) was measured to determine the antibody concentration, and the antibody was diluted in aseptic PBS (-) to 9.97 mg/mL. After dilution, filtration sterilization was performed through a φ 0.22 µm filter (Millipore).

The antibody was administered in the morning. The dummy cannula was removed from the mouse under ethereal anesthesia, an injection cannula (EICOM) charged with the antibody was mounted. After awakening, a 5-minute recovery period was given. Five minutes after, mouse IgG or P2L-1Ca (9.97 mg/mL) was intraventricularly administered (5 µL/2 min/mouse). After administration the animal was left for 5 minutes. The injection cannula was removed and then the dummy cannula was implanted under ethereal anesthesia. Approximately 24 hours after the antibody administration, saline (Otsuka Pharmaceutical) or MPTP (20 mg/kg) was intraperitoneally administered (5 mL/kg) 4 times every 2 hours. One week after the MPTP injection, the fourth MPTP injection was conducted and at the same time, PBS (-) was perfused through the left ventricle to remove the blood. Then, 1/15 M phosphate buffer (pH 7.4) containing 5% acrolein was perfused and the brain was excised. Thereafter, the brain was immersed and fixed overnight in 5% acrolein, which was replaced by 30% sucrose/PBS (-) on the following day. After replacement, the brain was frozen and embedded in Tissue-Tek compound (SAKURA) and stored at -80°C.

Tissue staining was performed as follows. A frontal section in 50 µm thickness was prepared using a cryostat and put in PBS (-). After rinsing 3 times with PBS (-) to wash off the compound, the section was treated with 0.5% sodium metaperiodate/PBS for 20 minutes to inactivate endogenous peroxidase. After rinsing 3 times with PBS (-), the section was treated with 1% sodium borohydride/PBS for 20 minutes to activate the antigen, followed by rinsing with PBS (-) and then blocking with TNBS for an hour. After rinsing with PBS (-), anti-TH antibody (Sigma), which is a marker for nigral and striatal dopamine neurons, was diluted to 8000-fold in TNBS and the reaction was carried out overnight. On the following day, after washing 4 times with PBS (-) for 5 minutes, biotinylated secondary antibody (Vector Lab.) diluted to 300-fold in TNBS was reacted for 2 hours, followed by washing 4 times with PBS (-) for 5 minutes. The ABC reaction was performed for 30 minutes. After washing 4 times with PBS (-) for 5 minutes, 0.02% DAB/ Tris-HCI (pH 7.6) supplemented with 0.006% H₂O₂ was added for color formation. After the reaction was terminated by rinsing with PBS (-), the section was attached to a slide glass and dried overnight on a spreader. On the following day, the section was dehydrated and penetrated by immersing twice in 100% ethanol (Wako Pure Chemical) and twice in xylene (Wako Pure Chemical) for 5 minutes, respectively. After sealing in MP500, the section was observed.

The number of TH-positive cells was determined by counting the cells in the substantia nigra pars compacta of -3.4 mm to -3.65 mm from the bregma twice in a blind manner. The mean values were used for analysis. The TH-positive fibers were quantified by analyzing the TH-positive fiber density in the striatum around -0.62 mm from the bregma using an image analysis software (Image-Pro Plus, MediaCybernetics).

Immunohistochemistry using the anti-TH antibody was performed on 3 mice in each group to determine the number of dopamine cells in the substantia nigra pars compacta and the striatal fiber density. As a result, a significant decrease of the dopamine cells in the substantia nigra pars compacta was noted in the group administered with normal mouse IgG and MPTP, when compared to the group administered with saline (P<0.001)(FIG. 4). In the striatum where dopamine nerve fibers are projected, a marked decrease of dopamine nerve fibers was noted in the dorsolateral site, and the results of image analysis showed a significant decrease when compared to the group administered with saline (P<0.05)(FIG. 5). On the other hand, the P2L-1Ca and MPTP group administered with the PrRP neutralizing antibody, significantly improved effects on dopamine cells (FIG. 4) and dopamine nerve fibers (FIG. 5) were noted as compared to the group administered with normal mouse IgG and MPTP.

### INDUSTRIAL APPLICABILITY

According to the screening method of the present invention, medicaments for the prevention/treatment of Parkinson's disease, etc., which have excellent effects and are free of side effects, can be screened.

## Claims

1. A method of screening a substance for the prevention/treatment of a disease selected from the group consisting of Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism, hypertension, burn, psychosomatic disorders and schizophrenia, which comprises using a protein comprising the same or substantially the same amino acid sequence as PrRP receptor and/or a protein comprising the same or substantially the same amino acid sequence as PrRP, or a salt thereof.

2. The screening method according to claim 1, which comprises using PrRP receptor and/or PrRP, or a salt thereof.

3. The screening method according to claim 1, which comprises using PrRP receptor or a salt thereof.

4. The screening method according to claim 1, wherein the disease is Parkinson's disease.

5. The screening method according to claim 1, wherein the disease is schizophrenia.

6. A method of preventing/treating a disease selected from the group consisting of Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism and psychosomatic disorders, which comprises inhibiting the binding of PrRP receptor and PrRP.

7. The preventing/treating method according to claim 6, wherein the disease is Parkinson's disease.

8. The preventing/treating method according to claim 6, wherein an antagonist to PrRP receptor is used.

9. The preventing/treating method according to claim 6, wherein an antibody to PrRP receptor is used.

10. The preventing/treating method according to claim 6, wherein an antibody to PrRP is used.

11. A method of preventing/treating a disease selected from the group consisting of Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism and psychosomatic disorders, which comprises inhibiting the expression of PrRP receptor and/or PrRP.

12. The preventing/treating method according to claim 11, wherein the disease is Parkinson's disease.

13. The preventing/treating method according to claim 11, wherein an antisense polynucleotide inhibiting the expression of PrRP receptor and/or PrRP is used.

14. The preventing/treating method according to claim 11, wherein a siRNA inhibiting the expression of PrRP receptor and/or PrRP is used.

15. An agent for the prevention/treatment of a disease selected from the group consisting of Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism and psychosomatic disorders, which comprises a substance inhibiting the binding of PrRP receptor and PrRP.

16. The agent for the prevention/treatment according to claim 15, wherein the disease is Parkinson's disease.

17. The agent for the prevention/treatment according to claim 15, wherein the substance inhibiting the binding of PrRP receptor and PrRP is an antagonist to PrRP receptor.

18. The agent for the prevention/treatment according to claim 15, wherein the substance inhibiting the binding of PrRP receptor and PrRP is an antibody to PrRP receptor.

19. The agent for the prevention/treatment according to claim 15, wherein the substance inhibiting the binding of PrRP receptor and PrRP is an antibody to PrRP.

20. An agent for the prevention/treatment of a disease selected from the group consisting of Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism and psychosomatic disorders, which comprises a substance inhibiting the expression of PrRP receptor and/or PrRP.

21. The agent for the prevention/treatment according to claim 20, wherein the disease is Parkinson's disease.

22. The agent for the prevention/treatment according to claim 20, wherein the substance inhibiting the expression of PrRP receptor and/or PrRP is an antisense polynucleotide.

23. The agent for the prevention/treatment according to claim 20, wherein the substance inhibiting the expression of PrRP receptor and/or PrRP is a siRNA.

24. A method of preventing/treating schizophrenia, which comprises promoting the binding of PrRP receptor and PrRP.

25. A method of preventing/treating schizophrenia, which comprises promoting the function of PrRP receptor.

26. The preventing/treating method according to claim 25, wherein an agonist to PrRP receptor is used.

27. An agent for the prevention/treatment of schizophrenia, which comprises a substance promoting the function of PrRP receptor.

28. The agent for the prevention/treatment according to claim 27, wherein the substance promoting the function of PrRP receptor is an agonist to PrRP receptor.

29. A method of diagnosis for a disease selected from the group consisting of Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism, hypertension, psychosomatic disorders and schizophrenia, which comprises assaying the expression level of a PrRP receptor gene and/or a PrRP gene, or an increase or decrease of the level of PrRP receptor and/or PrRP produced.

30. The method for diagnosis according to claim 29, wherein the disease is Parkinson's disease.

31. The method for diagnosis according to claim 29, wherein the disease is schizophrenia.

32. A kit for diagnosis of a disease selected from the group consisting of Parkinson's disease, motor function disorders, depression, neurosis, generalized anxiety disorders, Alzheimer's disease, hepatitis, hepatic cirrhosis, hepatic fibrosis, diabetes mellitus, chronic heart failure, acromegaly, hyperthyroidism, hypertension, psychosomatic disorders and schizophrenia, which comprises (1) an antibody to PrRP, an antibody to PrRP receptor, a PrRP gene or a PrRP receptor gene, and (2) a buffer.

33. The kit for diagnosis according to claim 32, wherein the disease is Parkinson's disease.

34. The kit for diagnosis according to claim 32, wherein the disease is schizophrenia.


SEQUENCE LISTING

<110> Takeda Pharmaceutical Company Limited

<120> Novel Use of PrRP and Receptor Thereof

<130> G06-0129

<150> JP 2004-268306
<151> 2004-09-15

<160> 10

<170> PatentIn version 3.3

<210> 1
<211> 87
<212> PRT
<213> Homo sapiens

<400> 1

Met Lys Val Leu Arg Ala Trp Leu Leu Cys Leu Leu Met Leu Gly Leu
1               5                   10                  15

Ala Leu Arg Gly Ala Ala Ser Arg Thr His Arg His Ser Met Glu Ile
            20                  25                  30

Arg Thr Pro Asp Ile Asn Pro Ala Trp Tyr Ala Ser Arg Gly Ile Arg

35                  40                  45

Pro Val Gly Arg Phe Gly Arg Arg Arg Ala Thr Leu Gly Asp Val Pro
    50                  55                  60

Lys Pro Gly Leu Arg Pro Arg Leu Thr Cys Phe Pro Leu Glu Gly Gly
65                  70                  75                  80

Ala Met Ser Ser Gln Asp Gly
                85

<210>  2
<211>  264
<212>  DNA
<213>  Homo sapiens

<400>  2
atgaaggtgc tgagggcctg gctcctgtgc ctgctgatgc tgggcctggc cctgcgggga      60
gctgcaagtc gtacccatcg gcactccatg gagatccgca cccctgacat caatcctgcc     120
tggtacgcca gtcgcgggat caggcctgtg ggccgcttcg gtcggaggag ggcaaccctg     180
ggggacgtcc ccaagcctgg cctgcgaccc cggctgacct gcttccccct ggaaggcggt     240
gctatgtcgt cccaggatgg ctga                                            264

<210>  3
<211>  82
<212>  PRT
<213>  Mus musculus

<400>  3

Ala Pro Arg Thr Trp Leu Leu Cys Leu Leu Leu Leu Gly Leu Val Leu
1               5                   10                  15

Pro Gly Ala Ser Ser Arg Ala His Gln His Ser Met Glu Thr Arg Thr
            20                  25                  30

Pro Asp Ile Asn Pro Ala Trp Tyr Thr Gly Arg Gly Ile Arg Pro Val
        35                  40                  45

Gly Arg Phe Gly Arg Arg Arg Ala Ala Leu Arg Asp Val Thr Gly Pro
    50                  55                  60

Gly Leu Arg Cys Arg Leu Ser Cys Phe Pro Leu Asp Gly Ser Ala Lys
65                  70                  75                  80

Phe Ser

<210> 4
<211> 12608
<212> DNA
<213> Mus musculus

<400> 4
gaattctaca cccagcacca atccaaatta agttcctgta ggtatcacat gctgagaggg      60
gtgtagccct caaacaccca gccagccctt ccctgccacc taggagataa gtgcttgggc     120
ctggaggccc tgagcccttt ccaggagaag cagtatctgt gtcctggagg cctctgctca     180
cactgtcctg tgtgttctat aaacccatgg acagaatgag accaagtaag atcatcgaaa     240

gcatctctta aattcctttc cccatctctg gcagagctgc agccaagagc agaaaccaca 300
gtgagcagaa gtagctgggc agctctgcag aaagcttcca cctaggagct cactaaacca 360
gccaagccct gcaagctcct cttcctcaag agacagaagg aagtcaaggc ccctgaacta 420
ccctcctagt aaataggctc tactgggaac ccacacgaat cgtggcctct catggtcatg 480
aactccacgt ttagaggaca gggcatcaca aggtagggat gcaagaaaag tggaagatac 540
tcatgagatc tctgtgagtt caaggacagc ctcctctacc taatgagttc cagaacagcc 600
agggctatgt aggaaagacc tgtctcaaaa aacaaaaaca aaaacaaaaa ctcaaccaaa 660
caaaggctgg tgaaaagcaa acacaagcaa ctgagttctg tgcacacatg aacccacagt 720
caggggcttg cctgtgtgcc tgtgtactgt gtgcactaac atggctgaga ttgtgtctat 780
ctgtatacat acccatattt ttcttcatac atatctatgt ctgtgtctat gcatgtaccc 840
atgtgtcacg tacatgctag tatgtgtgtc tgtgcatgca cacgtgggta tcagtgtaca 900
tacctgtgtg tgtgtctgtg tacacagcaa catgtctctc tgtatataca tatttctaca 960
tggcacttgt tggtgtgtat atctatgtgt gcatgcacac atgtatatac atagatctaa 1020
gcttgtatct gtctccccac ctgtgtgtgt atacacatat acatacatac atatatatat 1080
atatgtgtgt gtgtgtgtgt gtgtgtgtgt gtgtgtgtgt gtgtttctct gttatatctg 1140
tatctgtcat ataattgtta gtatatttgt ttacttctgt gcaaacactt tgcccatgaa 1200
cctatgtgca tgcctaaacg aacatgtgta tttgtgccct acagaaaccc gtgatggccc 1260
agcagcccta aagggaggct catggcacag gacagtacca caaagcaccc cacagtttcc 1320
tccacatgac cgtcgcctcc ccatgggctc tgttctttgt gatgctcaca gctgcttgta 1380
gtcatcattc cagtgaggag taagaagaat tttatgtgtg tctctacctg tgagactgca 1440
gaatctcaat cctccagctg tcaccaggtc accaaccagg cacctgccag cctctgacct 1500
gggaaataca aactctgtga cttctcctca actgatgttc tctgcctact gaaggtcact 1560
cagaagccat ctcgctgtga ttctttaacc cgatcctcga ctttgctggg ccaccctcac 1620
agctgggatg aaaagagctg gttcagagga agccctgaac atgtacacat ccatgactat 1680
gcacgagcat gagagagaga gagagagaga gagagagaga gagagagaga gaggaagaga 1740
gagagagaga gaatgaatct gtgggcatga tgagagagca tacaattgca ggtggtgaat 1800
gggggagggg gtggattctt cccctacctg acagattttg ttcttcaaac ttccttgtgt 1860
gttctagatg agggcagata acactgacca cccccacccc agcattccca ccacaataac 1920
cactatccac aatgccacac taagaccaag cctccaacac aggcttttgg ggtacactac 1980

atggggacct cgctttctat atagcttgtc ttcaaatgtc ggcttctaac acacacagat 2040
gactgggatt tatcaagaac tcagatcttc caggtcaagc ccgtcagagc cagttttgct 2100
ggtgtctgca cggtctgccc ttcacagcca tcccaccact acagcggggc tgctacccac 2160
acccagcgct gcctctttcc tgttgtcggt ttgtatcctg ttctgggcta gagaacacac 2220
aggccagagc cacaaacatc ccccgtgtct tctgaaaata aactccagga ggaagcctct 2280
cactcattca gagctcctct caggagcgcc atatttgtta caacctgatg caggaaatgt 2340
gtcctcctgt gtcacctgtg ccctgaactg cttgccacct gaccctacct gcccttgggg 2400
gttggaggtg aggtggcgca gagtcaacaa cacagactcc gggaggtggt gggaaacaga 2460
aacgctgcag caagttcatc tgaacaatgc tctttaatac cctccaccca agccccattg 2520
gtcccaggaa agcacctcct cctcctcctc ttcctcctcc tcctcctcct cctcctcttc 2580
ctcttcctcc tcctcttcct cctcctcttc ctcctcctcc tcttcctctt cctcttcctc 2640
ctcctcttcc tcctcctcct cctcctcttc ctcctcctcc tcctcctcct cttcctcctc 2700
ttcctcctcc tcctcctcct cttcctcctc ttcctcctcc tcctcctcct cctcctcttc 2760
ctcctcctcc accttgttct agagttcctt accataccca cctcccaagg agcccactgg 2820
ccagagccct cttccaagga agaggcagcc attccttgag aacccagatg ctacgctgac 2880
cacgtgtcca aaggatgacg gggcagccta aagcttttct acaagtctgg tggtgggcag 2940
gaaatggtga tcctggggag acacctgcct ggtctcttct cgtgcacagc tccttcaaga 3000
atgttctatt gcacctcaat gatatttcat ataacctgta cctattgtcg tggtgatgac 3060
ggggtgagtt aaatggcaga acgttgacag tcctttcacc agcaacagac ctgcagatac 3120
ttcaacactt ccagaatata tgctggtgaa agaatgaggc tcagcacctc taagcgggct 3180
caggagtctt cccagaaggc acaccccgaa aggcaaacac tgacctttcc aggtgatctg 3240
agctcttcta tctatttttg tgtgcactaa ttctttctca ttacatgtaa attacctttc 3300
cagcataata gacttccttt tttaaaagat ttttttgcct gcgtgtatgg ggtgaggtgt 3360
gtacacatct atgaggggac gctgtgcact tgtgcataga gaccagagat taatgtggtg 3420
tcttcctcag tcatgtctcc atttcctgtt tttgagacac tgatcctgga ggttgtcagt 3480
ttggctatgc tgactggcca gtgagcatca ggggtcttcc acacctctgc tctctctacc 3540
ttagcactgg ggttataaac cggcgctgcc atgttctgcc tttaaaccta ccttacaata 3600
aaagtaacac tctgtttcgt tttcattttt ttattgtttt tctttattga ggcagcctgg 3660
ttttgaactc atagccatcc tcctgcttca cttcccagag agctggggtt acagtcatgt 3720

accacaatgc caggcttctg attgtattgt atcaataaac ttggtgtgtt ctcagccata 3780
gactcaggat gaaccagaga aaatgtgtct agagaccgtg catgtggata agcaagctga 3840
ggggacaacc agagtaggag gtacccacct gggttcaagg gacttcctgg ccacaattcc 3900
aggaagtcat ggctcaggtg ggacaagtat tcagaagagg ccatattcta gttcacattg 3960
agaggacggg tggacacgtc actgtggagt ctgcccaaag gcgagcgtga agagggggca 4020
gggggggtgt taatgtgttt taaagccact tggctcaaat gaaattatat aattcaggaa 4080
ggaacaaacc acgtcagtga tgagaggttt gggttttttt tttttttaag aaggtaaaaa 4140
aagaaaggaa aggaaaaaaa agacggagtg tcttgatgag atgatgagag aagagacgta 4200
gtggaaactg caggctataa gcaaagacgc catctcaagg aggggtctgg gggcgtcctt 4260
tgcctccttg ttggcctata aaagataagc aggcaggcag acctcacttc acgcccaggc 4320
agccttatcg ccacactcaa gggacagaga gagggtgagt aacttggcag tgctggagag 4380
ggctgggtgt tgctgtgact tccagcttct ccacaagtgt tttctctgcc aggcttactg 4440
ggcagccaag tgcgggcacc aagcaggaca agcagagaga actacctgtg gtctgtggtc 4500
taggaccaca caccattctg gggtctctga cactcaaggg ctgtccatga tggagggcag 4560
gtgaaggtgg ccctcagtgg gacacctcaa gcaaagccca gaaggcaccc tccagaaagg 4620
ctcagaactc aggatcagtt agcattctca gtccccatca cccagcctga gtgggtgcag 4680
tctttacaat gattcatggt ggctcctcca ttcctctagt tagggttccc cttcactgtg 4740
tgggacatcc caggaggagg aggaggagag ggtgggcatt taaagagctc tgatggacag 4800
cgctgtgtgg acaaggagaa gctggagcgt caaagtcagc tggggggggg gggtcttcct 4860
tgagcatctt ctaataggac ctgaaatgtc aggggtagga agaaaagggt cccctgcagg 4920
aagtatcaca ggtggatgct atgtggtagg tcagaacact ccatcagcag ggtaccagtt 4980
cccatacaca gtggcggcag ccttcccatg tcattcaggg ggactctgtg aggagaggcc 5040
cattctggag tctcagcaaa ctgagtctaa agtcctgggt tattgtagag gctgtgaggt 5100
gtgagctctc tggggatgtg ggatgctaag tgtcctccat tgcctgcctg gctctcagtg 5160
tcaggagatc ccagacaaag tgtgaggaaa gcctgggcac tgaggttcca atatgttcca 5220
actcctagct acctctgagg ttgggcaaat tgttctccac ctctgctgac cacaaggctg 5280
cccataaatc tgcttcccac caaggcctgg gtgtctacct cccagctctt gacacagatg 5340
gacagacaga cccaaggatg tcccaagaca gccacctgtg cacagctcac acctctcccc 5400
tgataattgt agctatgtgc actaaacata tgcatttgca caccttatag gcacggacac 5460

gcaccacaca cacaagtaca tttgtacaaa caacctaggg tcccttctgg ctttgtgcat 5520
acaacgtact ttgcattttt acccccaggc ttcaggatcc aattttcagg gcatcattca 5580
ggaagccgga agcatggcac cgaggacctg gcttctgtgc ttgctgctgc taggcttagt 5640
cctcccagga gcttccagcc gagcccacca gcactccatg gagacccgca gtgagtgcct 5700
ggcatatgga ggacagccac tgtcacctcc catccatatg cttcccaaat gccttgagta 5760
cccagcccct gaatgggagg ttagccatct cctaagccag tggtttccaa ccttcctaat 5820
acagaacttt taatacagat ccttatgttg tggtgacccc cagccagaaa attattgtga 5880
tgctgttttc atagttgtaa gttttgctac tgttatggat cataatgtta atatctgaaa 5940
tgcaggatgt ctgatatgcg cccttccccc caaacaaaag ggacacaacc cacaggttga 6000
gagcctctgg gatctaagca aaagctacct taccatgcag tcagttggga gattggtcct 6060
gttaagatct ccccagaatg gtcctgtttc ctgtcctcat cattccccta acccatcttt 6120
gtggggtccc ttaagacttt ggaggatgac agtcagacag gaagagaata ctgatcctgg 6180
catatgtcta aataaattcc ctaaagccac accactgccc agatatgccc agccagtgta 6240
atcagggtgg gtgccaacat ggcctggtgc ccaggtttcc atcagcttag gggctcccgt 6300
gtcccatacg ctgctctgac tctttccttt ccagcccctg acatcaatcc tgcctggtac 6360
acgggtcgtg ggatcaggcc tgtgggccgc ttcgggagga ggagggcagc cctgagggat 6420
gtcaccggac ctggcctgcg gtgccggcta agctgcttcc cactggatgg aagtgccaag 6480
ttctctcaca gctcatgaag acagtgatgc tgagcccaag cccaccctcc ctgtcccccc 6540
gcagagcctc ctccaccctc cctttccggt tttccctctg atctaataaa agtgctggct 6600
ttgtttattg tacacttgta actatgtggt aacaaaccgg aaggtgcttt ctctctgggg 6660
agggtaacca tgaaagaagc tcagaaccca gtaacctctt tggaaagaag aagctcccac 6720
ctgcccccaa tagaacaact gagatcgctc attaccaggc cccacagaag ttgtcctggt 6780
cccttaagac cctgcagtgg gggaagggaa tgttgattca gtgttcctat aaattcctgt 6840
tccccaaaac cacatcactg cccagaaatg gggtgccctt ttcttggtca cttgttccca 6900
caagcttcca cctacacaag gtaccctgaa cacatgctca gcttgcatac tgcataaagc 6960
cagggtaacc acacactaca ctatgtaaga tacttggtcc cagagaaaag ccacacaacg 7020
cccctggcat ggctcacgtc cttctcttgg catttttggg tttgtttttt tttttgtttt 7080
tgtttttgtt tttttgtttt tttgttttgt tttgttttgt ttttgttttt ttgttttttt 7140
tttgtttttt tggtttttcg agacagggtt tctctgtaaa gccctggctg tcctggaact 7200

cactttgtag accaggctgg ccttgaactc agaaatctgc ctgcctctgc ttcccaagtg 7260
ctgggattaa aggcatgcgc caccacgccc ggcagcattt ttaagattaa catttagaga 7320
tgtttagggg tttgctgcaa aaattagatg ctatcaaagt gttgttccta gcacccagaa 7380
cagagaacat tcattacagc ctatgaagcc accaagtgtc atcgcacagg acctatgcta 7440
tccataggtg catgttcggc attgtgtctg ctgtttgggc aaatgtatta cgtggctcca 7500
ccagttcagt gacaatgtgg tgcccctcat caccctgaac acaccccatt ccttgtccct 7560
tgcagaatcc tgcagctacc aacacacatg ggtatgttaa cctacttcct gctcagagga 7620
gaatcccaga gacttgaccc cattcttttt gatcctatag gcctccttta aagtcacttg 7680
gggtgtccta tgccctttgt caaacacaaa gtgactggca cataacacag ctggtgctcc 7740
cagaaaaggg gggaaagtca gacaggagct gagggagacg ccgtgggaag ccggggcagc 7800
tgaaattcac ctgcacagag gagtgccaat ggctgccagc agcctcaccc accagcacag 7860
aagcctgaac tggcctcttc tcagctcagt aggtgccatc ttaccctcag atcaagatga 7920
tctcagctgg gggtcatctg gctggctgct gtctatgctg ttcccctcag gttaccctca 7980
ggctgctgtg actgttgtca caggccgcta tgactgttgt cacaggccgc tatgactgtt 8040
gtcacaggcc gctatgactg ttgtcacagg ccaactcccc tccagttctt tctactccct 8100
gtatacttca gcaccttcca acgccacttt ttcctggggt ggtgacatgt ggctgcggtg 8160
aaaggcataa gtggggattg gctgggatct cagatgaagg tctcatgacc ttacccatcc 8220
ctcctcccgg gcaggaacac aggctggctc ttgttgatgg tttcctgcag gcaatagctg 8280
ctgctctgac agcagtggta atggctgcca tgcttggagg acatcctccc acagctctta 8340
tccagaaaga acagctcctc gtctggaaag atgaatggag gcgtactcat cttttccaca 8400
gtctgggaac ggttttaaaa catacagaaa agttcacaag acattgtaag gactatctca 8460
acctacatgg actcatcagc catcctgtct gcctgcttcc tacttcccat ccttgccaac 8520
ctacatttga tgacaagctg cagacaccaa gacattgcct caaagagttc aaacctttct 8580
cccaagaata acacaaagtg taagcctgga aaagtctatt ctctctctct ccctctctcc 8640
cctctctctc cccctctcct ccccctcccc tctccctctc cctcttctcc ccctctctct 8700
ctgcatgtgt gcatgtgtac gtgtgtgtgc atgcatgtgc atgtgtgtgt gtgcacacac 8760
gtgtgtgcat gtgctcatat gtgcatatgt acgtgtgtgt gtgtgtgtgt gtgtgtgtgt 8820
gtgtgtgtat gtgtctcagc ccctgatctt acatcataac atcaaccttc tgaagcaccc 8880
agtggcctgt tctttaaaat gatccttgct gattatccca cctagtggtg agcctgacac 8940

tgctcttgca cttcctgtca cagaaaagga cctgggcctt agctggacat tatactctgc 9000
tgtccaaaag tcctgggtcc tctgatggag tcctgggtcc cactttgaca tgttgacctt 9060
gaccttgagt tcagggccct gcctctcaac tgtaagagat ctctccatcc ctgggcacca 9120
ctgactttca caagcctggt gaagctgggt ggttccagat ccatccagtc ttgggctttc 9180
tgctggggtg tgtcaaaggg agcattacta atcagtcaag agaaaggaga gattggtaac 9240
cagacaggca gtcacctaca aatcttcctg ctgtatccct gcttcctcaa agcagtgatt 9300
ttgccctgaa gagatgcacc ggagtgtcta ggtacccagg acccaacaga aggatttgtc 9360
ttaagtgata ctagatcgcc acctagtggc catatgtaac ccggcctctc ttccccctct 9420
agggtaggtt gcctacctgc ctaatgattc ccagccctta gggatgatga ggacagcccg 9480
ggatgctgaa tattcacttc ccctgtttca catgtgagcg agtcaccagg tggcaacaat 9540
gtatcttccc tagctgtcag aaaagcagtt gcccagcatc aacaggcctc actaagaaac 9600
ccaccaggga agagggagtg aaaacctccc acacggccct tgtcatctgc tcactgatcc 9660
tgacgtgaac agtgtctggc tcacatttgt ccagtggtca cagggtttcc ttcaggtcat 9720
gtcaggacta catcagtgcc tagaaactca caatcgtttc caggccccag aacggaatga 9780
agctgcatcc ctctagaagt gcagcggggc cagaaagttc cctccaatcc ctggcctgag 9840
ccccatgtga aacttgaagg gagaaagcac tggtgtggtc ctgtccaagc aaagccaatg 9900
ggagcaggag tcacagccca ggcacagaca agctcctggc cagcctgggg acacgttcac 9960
ttttatccat ttgagcttca cagtgggcac ggtgaagcca tcttccttgg tgagataaca 10020
atgttccact ctgtgatgtt ctctagggct tgtttgactc tgttggtttt gttgtttgct 10080
tgtttgtttg tttgtttgtt ttctcactgt gcagcccaga ataagctcgc cattctcctg 10140
catcagcttc acaagtgtcg ggattcgagg cttacatcac cacacccaac aacaatgtct 10200
cattgttcag actcaacaga taatcacact cccagaagca aggattagca aggatttaca 10260
cacacacaca cacacacaca cacacacaga gagagagaga gagagagaga gagagagaga 10320
gagagagaga gctttgctcc ctaaatggcc acaccttgcc ccagcaagtg tcaagagaga 10380
aacttaacag gaagatgttt tctggggctt tcttcccctg ctcaaggacc tgagtttctc 10440
tacaaggata agaactcaca caatcaccat cagaagaact cacagtccac cccagaccaa 10500
tgcctgtgcc caccctcgtt acactcaccc ccatgaaccc tcaccatgtt taacacccac 10560
aaacccatct agaacgtgtg cagaaaaact gaacaggtgt gacccgtcgg gccatgggtc 10620
cgaatataac ctcatgaagt gttcggattg tcaagacgtt taatatttgg agagaaggac 10680

atgagacaac acggtgttca tctcgggaca ggggttttag agtcctcaga tcactaggta 10740
gaaacctggc tctgtccatc caccgctgga agggggacac ttccatccca gccacccttt 10800
cagactctgt ctctcctgcg tcacctcact gagacttagc accttgggct cgtttttctc 10860
ttgggctcat ctctgcattt ttccttgcat tgatgcccct tctctccccc tgacagttcc 10920
cagaacgaaa gtggtccagt tatggatgct gccccaggag gaagcatctg tcctcccatc 10980
ccacggatca aaggcttttc ccagtccatg ctgcttctct gcttcattta gctctctgtg 11040
ctggtcacaa ggaagctgaa agggacagcc caggaggagc aggcacactt ggctcagggc 11100
atccaagtat ctgggtatca ggtaaggtag ggctgacagt cttctgggag ccatttgaca 11160
ctggatcttc cctgagacag ttctcatcag ctttggcaag cagtgattac agagaaaacc 11220
aacgcctgga agcctccagg gctcaagcat aagccatctc tgacacagaa catgaatgga 11280
caaagaggca gccccggcag atttgccaca aagacgtagt agcttgggct ggggaccctc 11340
tgaagcacag gctgagatgt gtcctccgcc agctctcctg cagtggcagc ggctatcgtg 11400
cacagcactg gcgctgcctg atgggaggtt cctggttcag agccacagcc tcaccttcct 11460
gtgggcggct gctccccgtg tctcctgctc tctgcagaag ctcctgagct gtggaaaaca 11520
gggaggtgta ctggagagca ccacccagaa ccctaaccaa acattcccta ttagtcttcc 11580
tcatacccct ccctgcccca aggacaccag aagtcaaccc aagggtggag gcatagcagc 11640
gagctctcac actccctgtg gaggggccgt aactcaccaa cagtgttgaa gatctcagac 11700
acctggtagt tcagcttggc ggaggtttcc atgaacagca agcttttgct ctctgcaaac 11760
tcttttcctt cctgaaggaa aaagtcagtg tgtttaatga gagcccctcc aaagactcac 11820
taagtccaac gactgtggtt gactcacaag aaatggaaat gcacctgtaa tcccagcact 11880
aaagaagctg gggcagaagg attatgagtt caaggtcagc cttggacaca tacgtccctg 11940
cctcaaaaca atagaaaaag acatctatgt gcacatattc ttcacacaga ccacctgata 12000
tacacacact ctctgtacat atgtgtacat acaccttcta tatgaatctc caactagaag 12060
aggaactaag atggaatctt tgtgcatttc tatacacaca agtgcatctg catgtgggtt 12120
tgcatgtggt gactttggag gcacatgtgt gtgttcatga atatatatcc atgaagaacg 12180
actctgggca tgtgcatgag catgtgtgtt tatgaattat gaggtagaga gatcatgggt 12240
atacatgtaa gtgcaagtat atggatgagt atgtgagtgc ctgaggcaca gaggtatatg 12300
ctgtgtgtgt tgtgtgtgtg tgtgtgtgtg tgtgtgtgtg tgtgtgtgtg tgtatgtgtg 12360
agagagagag aaagagagag agagagagag agagagagag agagagagag agagtatgtg 12420

tgtgtgtgtg tgtgtgtgtg tgtgtgtgtg tgtgtttcca ggcctcaaaa agtacttgag 12480

tccacagaat caagaactcc ctcctgctgc ccgccaccat gcggcacctg gaaggtcact 12540

tcccgctcct cgcccagatc cgttttgttg ccgaccagca tcaccaccac ctctcctggc 12600

tggaattc 12608

<210> 5

<211> 83

<212> PRT

<213> Rattus norvegicus

<400> 5

Met Ala Leu Lys Thr Trp Leu Leu Cys Leu Leu Leu Leu Ser Leu Val
1               5                   10                  15

Leu Pro Gly Ala Ser Ser Arg Ala His Gln His Ser Met Glu Thr Arg
            20                  25                  30

Thr Pro Asp Ile Asn Pro Ala Trp Tyr Thr Gly Arg Gly Ile Arg Pro
        35                  40                  45

Val Gly Arg Phe Gly Arg Arg Arg Ala Thr Pro Arg Asp Val Thr Gly
    50                  55                  60

Leu Gly Gln Leu Ser Cys Leu Pro Leu Asp Gly Arg Thr Lys Phe Ser
65                  70                  75                  80

Gln Arg Gly

<210> 6

<211> 252

<212> DNA

<213> Rattus norvegicus

<400> 6

atggccctga agacgtggct tctgtgcttg ctgctgctaa gcttggtcct cccaggggct 60

tccagccgag cccaccagca ctccatggag acaagaaccc ctgatatcaa tcctgcctgg 120

tacacgggcc gcgggatcag gcctgtgggc cgcttcggca ggagaagggc aaccccgagg 180

gatgtcactg gacttggcca actcagctgc ctcccactgg atggacgcac caagttctct 240

cagcgtggat aa 252

<210> 7

<211> 98

<212> PRT

<213> Bos taurus

<400> 7

Met Lys Ala Val Gly Ala Trp Leu Leu Cys Leu Leu Leu Leu Gly Leu

1               5                   10                  15

Ala Leu Gln Gly Ala Ala Ser Arg Ala His Gln His Ser Met Glu Ile

            20                  25                  30

Arg Thr Pro Asp Ile Asn Pro Ala Trp Tyr Ala Gly Arg Gly Ile Arg

        35                  40                  45

Pro Val Gly Arg Phe Gly Arg Arg Arg Ala Ala Pro Gly Asp Gly Pro
50                  55                  60

Arg Pro Gly Pro Arg Arg Val Pro Ala Cys Phe Arg Leu Glu Gly Gly
65                  70                  75                  80

Ala Glu Pro Ser Arg Ala Leu Pro Gly Arg Leu Thr Ala Gln Leu Val
                85                  90                  95

Gln Glu

<210> 8
<211> 297
<212> DNA
<213> Bos taurus

<400> 8
atgaaggcgg tgggggcctg gctcctctgc ctgctgctgc tgggcctggc cctgcagggg      60
gctgccagca gagcccacca gcactccatg gagatccgca cccccgacat caaccctgcc     120
tggtacgcgg gccgtgggat ccggcccgtg ggccgcttcg gccggcgaag agctgccccg     180
ggggacggac ccaggcctgg cccccggcgt gtgccggcct gcttccgcct ggaaggcggc     240
gctgagccct cccgagccct cccggggcgg ctgacggccc agctggtcca ggaataa        297

<210> 9
<211> 370
<212> PRT
<213> Homo sapiens

<400> 9

Met Ala Ser Ser Thr Thr Arg Gly Pro Arg Val Ser Asp Leu Phe Ser
1               5                   10                  15

Gly Leu Pro Pro Ala Val Thr Thr Pro Ala Asn Gln Ser Ala Glu Ala
            20                  25                  30

Ser Ala Gly Asn Gly Ser Val Ala Gly Ala Asp Ala Pro Ala Val Thr
        35                  40                  45

Pro Phe Gln Ser Leu Gln Leu Val His Gln Leu Lys Gly Leu Ile Val
    50                  55                  60

Leu Leu Tyr Ser Val Val Val Val Val Gly Leu Val Gly Asn Cys Leu
65                  70                  75                  80

Leu Val Leu Val Ile Ala Arg Val Arg Arg Leu His Asn Val Thr Asn
                85                  90                  95

Phe Leu Ile Gly Asn Leu Ala Leu Ser Asp Val Leu Met Cys Thr Ala
            100                 105                 110

Cys Val Pro Leu Thr Leu Ala Tyr Ala Phe Glu Pro Arg Gly Trp Val
        115                 120                 125

Phe Gly Gly Gly Leu Cys His Leu Val Phe Phe Leu Gln Pro Val Thr
    130                 135                 140

Val Tyr Val Ser Val Phe Thr Leu Thr Thr Ile Ala Val Asp Arg Tyr
145                 150                 155                 160

Val Val Leu Val His Pro Leu Arg Arg Arg Ile Ser Leu Arg Leu Ser
                165                 170                 175

Ala Tyr Ala Val Leu Ala Ile Trp Ala Leu Ser Ala Val Leu Ala Leu
            180                 185                 190

Pro Ala Ala Val His Thr Tyr His Val Glu Leu Lys Pro His Asp Val
        195                 200                 205

Arg Leu Cys Glu Glu Phe Trp Gly Ser Gln Glu Arg Gln Arg Gln Leu
    210                 215                 220

Tyr Ala Trp Gly Leu Leu Leu Val Thr Tyr Leu Leu Pro Leu Leu Val
225                 230                 235                 240

Ile Leu Leu Ser Tyr Val Arg Val Ser Val Lys Leu Arg Asn Arg Val
                245                 250                 255

Val Pro Gly Cys Val Thr Gln Ser Gln Ala Asp Trp Asp Arg Ala Arg
            260                 265                 270

Arg Arg Arg Thr Phe Cys Leu Leu Val Val Val Val Val Val Phe Ala
        275                 280                 285

Val Cys Trp Leu Pro Leu His Val Phe Asn Leu Leu Arg Asp Leu Asp

290                 295                 300

Pro His Ala Ile Asp Pro Tyr Ala Phe Gly Leu Val Gln Leu Leu Cys
305                 310                 315                 320

His Trp Leu Ala Met Ser Ser Ala Cys Tyr Asn Pro Phe Ile Tyr Ala
                325                 330                 335

Trp Leu His Asp Ser Phe Arg Glu Glu Leu Arg Lys Leu Leu Val Ala
            340                 345                 350

Trp Pro Arg Lys Ile Ala Pro His Gly Gln Asn Met Thr Val Ser Val
        355                 360                 365

Val Ile
    370

<210>  10
<211>  1113
<212>  DNA
<213>  Homo sapiens

<400>  10
   atggcctcat cgaccactcg gggccccagg gtttctgact tattttctgg gctgccgccg      60
   gcggtcacaa ctcccgccaa ccagagcgca gaggcctcgg cgggcaacgg gtcggtggct     120
   ggcgcggacg ctccagccgt cacgcccttc cagagcctgc agctggtgca tcagctgaag     180
   gggctgatcg tgctgctcta cagcgtcgtg gtggtcgtgg ggctggtggg caactgcctg     240
   ctggtgctgg tgatcgcgcg ggtgcgccgg ctgcacaacg tgacgaactt cctcatcggc     300
   aacctggcct tgtccgacgt gctcatgtgc accgcctgcg tgccgctcac gctggcctat     360
   gccttcgagc cacgcggctg ggtgttcggc ggcggcctgt gccacctggt cttcttcctg 420
   cagccggtca ccgtctatgt gtcggtgttc acgctcacca ccatcgcagt ggaccgctac 480
   gtcgtgctgg tgcacccgct gaggcggcgc atctcgctgc gcctcagcgc ctacgctgtg 540
   ctggccatct gggcgctgtc cgcggtgctg gcgctgcccg ccgccgtgca cacctatcac 600
   gtggagctca agccgcacga cgtgcgcctc tgcgaggagt tctggggctc ccaggagcgc 660
   cagcgccagc tctacgcctg ggggctgctg ctggtcacct acctgctccc tctgctggtc 720
   atcctcctgt cttacgtccg ggtgtcagtg aagctccgca accgcgtggt gccgggctgc 780
   gtgacccaga gccaggccga ctgggaccgc gctcggcgcc ggcgcacctt ctgcttgctg 840
   gtggtggtcg tggtggtgtt cgccgtctgc tggctgccgc tgcacgtctt caacctgctg 900
   cgggacctcg acccccacgc catcgaccct tacgcctttg ggctggtgca gctgctctgc 960
   cactggctcg ccatgagttc ggcctgctac aaccccttca tctacgcctg gctgcacgac 1020
   agcttccgcg aggagctgcg caaactgttg gtcgcttggc cccgcaagat agccccccat 1080
   ggccagaata tgaccgtcag cgtggtcatc tga 1113